# EUROPEAN PATENT SPECIFICATION

(11) **EP 2 118 276 B1**
(45) Date of publication and mention of the grant of the patent: **29.07.2015**
(21) Application number: 08725297.9
(22) Date of filing: 06.02.2008
(51) Int. Cl.: C12P 7/06, C12P 19/14

(54) **STARCH HYDROLYSIS USING PHYTASE WITH AN ALPHA AMYLASE**
STÄRKEHYDROLYSE MIT HILFE EINER PHYTASE MIT EINER ALPHA-AMYLASE
HYDROLYSE D'AMIDON EN UTILISANT UNE PHYTASE AVEC UNE ALPHA-AMYLASE

(30) Priority: 07.02.2007 US 900237 P; 06.03.2007 US 905222 P; 06.03.2007 US 714487
(43) Date of publication of application: 18.11.2009
(73) Proprietor: Danisco US Inc., Palo Alto, CA 94304 (US)
(72) Inventor: PAULSON, Bradley, Alan, Palo Alto California 94304 (US); POWER, Scott, D., Palo Alto California 94304 (US); RAMER, Sandra, W., Palo Alto California 94304 (US); SHETTY, Jayarama, K., Palo Alto California 94304 (US); WARD, Donald, E., Palo Alto California 94304 (US)
(74) Representative: Forrest, Graham Robert
(86) International application number: PCT/US2008/001647
(87) International publication number: WO 2008/097620

(56) References cited:
- WO-A-01/62947
- WO-A-02/38787
- WO-A-02/092797
- WO-A-2005/111203
- WO-A-2006/043178
- WO-A-2007/044968
- WO-A-2008/021050
- WO-A1-96/28567
- US-A- 5 866 118
- COWIESON A J ET AL: "Carbohydrases, protease, and phytase have an additive beneficial effect in nutritionally marginal diets for broiler chicks", POULTRY SCIENCE, vol. 84, no. 12, December 2005 (2005-12), pages 1860-1867, ISSN: 0032-5791

## Description

### FIELD OF THE INVENTION

The present invention relates to enzyme compositions comprising at least one phytase and at least one alpha amylase, methods for liquefying starch, and methods for the production of end-products, such as glucose and alcohols (*e.g*., ethanol).

### BACKGROUND OF THE INVENTION

Industrial fermentation predominantly uses glucose as a feedstock for the production of a multitude of end products such as enzymes, proteins, amino acids, organic acids, sugar alcohols, pharmaceuticals and other biochemicals. In many applications glucose is produced from the enzymatic conversion of substrates comprising starch and cellulose *(e.g.* whole milled cereal grains). Starch, which comprises two polysaccharide fractions, amylose and amylopectin is deposited in plant cells as granular particles. The partial crystalline structure of these granules imparts insolubility in cold water, and as a result, solubilization of starch granules in water generally requires heat energy to disrupt the crystalline structure of the granule. Numerous processes have been employed for starch solubilization and these include direct and indirect heating of substrates comprising granular starch. (See, for example, STARCH CHEMISTRY AND TECHNOLOGY, Eds R.L. Whistler et al., 2nd Ed., 1984 Academic Press Inc., Orlando FL and STARCH CONVERSION TECHNOLOGY, Eds G.M.A. Van Beynum et al., Food Science and Technology Series 1985 Marcel Dekker Inc. NY).

Starch to glucose processing generally consists of two steps and these steps include liquefaction of starch and saccharification of the liquefied starch. Further steps may include (a) purification and isomerization when the desired end product is a purified dextrose or fructose or (b) fermentation and distillation when the desired end product is, for example an alcohol *(e.g.,* ethanol).

An object of the starch liquefaction process is to convert a slurry of starch polymer granules into a solution of shorter chain length dextrins of low viscosity. This is an important step for convenient handling of industrial equipment used in starch conversion processes. Commonly, the starch is liquefied by use of high temperature and enzymatic bioconversion. For example, a common enzymatic liquefaction process involves adding a thermostable bacterial alpha amylase (*e.g*. SPEZYME® PRIME and SPEZYME® FRED, SPEZYME® XTRA (Genencor International Inc.) or TERMAMYL SC, TERMAMYL SUPRA or TERMAMYL 120L (Novozymes)) to a slurry comprising a substrate including granular starch and adjusting the pH to between 5.5 to 6.5 and the temperature to greater than 90°C. The starch is gelatinized and then can be subject to saccharifying enzymes. Typically, saccharification takes place in the presence of glucoamylase enzymes such as glucoamylase from *Aspergillus niger (e.g.* OPTIDEX L-400 (Genencor International Inc.)) at a pH more acidic than the pH of the liquefaction step. The pH of a typical saccharification step is around pH 4.0 to 5.0.

WO2006/043178 describes the cloning of a phytase gene from Buttiauxella sp. P1-29.

WO96/28567 describes the use of phytase to improve starch liquefaction. WO01/62947 describes fermentation of material containing phytic acid in the presence of phytase.

A number of variations exist for the liquefaction and saccharification of a starch substrate and despite advances made in the prior art, a need still exists for more efficient means for starch liquefaction.

### SUMMARY OF THE INVENTION

The invention provides method for liquefying starch, said method comprising:
(a) incubating a slurry comprising a granular starch substrate with an enzyme composition, at a temperature which is above 55°C and below the initial starch gelatinization temperature of the granular starch substrate, for about 2 mins to about 4 hrs at a pH range of about 4.0 to about 6.2;
(b) raising the temperature to 0 to about 45°C above the initial starch gelatinization temperature for about 5 mins to about 6 hrs at a pH of between about pH 4.0 and about 6.2 and obtaining liquefied starch;
   wherein the enzyme composition comprises a mixture of a phytase and an alpha amylase, wherein the phytase has an amino acid sequence having at least 90% sequence identity to the sequence BP-WT: 
NDTPASGYQV EKVVILSRHG VRAPTKMTQT MRDVTPNTWP EWPVKLGYIT
PRGEHLISLM GGFYRQKFQQ QGILSQGSCP TPNSIYVWAD VDQRTLKTGE
AFLAGLAPQC GLTIHHQQNL EKADPLFHPV KAGTCSMDKT QVQQAVEKEA
QTPIDNLNQH YIPFLALMNT TLNFSTSAWC QKHSADKSCD LGLSMPSKLS
IKDNGNKVAL DGAIGLSSTL AEIFLLEYAQ GMPQAAWGNI HSEQEWASLL
KLHNVQFDLM ARTPYIARHN GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 or to the sequence BP-11: 
NDTPASGYQV EKVVILSRHG VRAPTKMTQT MRDVTPNTWP EWPVKLGYIT
PRGEHLISLM GGFYRQKFQQ QGILSQGSCP TPNSIYVWTD VDQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 or to the sequence BP-17:

NDTPASGYQV EKVVILSRHG VRAPTKMTQT MRDVTPNTWP EWPVKLGYIT
PRGEHLISLM GGFYRQKFQQ QGILSQGSCP TPNSIYVWTD VAQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ.

The alpha amylase may be from a *Bacillus sp.,* e.g. *Bacillus stearothermophilus* or *Bacillus licheniformis.*

The composition may be a starch hydrolyzing composition.

The phytase may have, for example, at least 95% sequence identity to the sequence BP-WT, BP-11 or BP-17, or may have the sequence of BP-11 or BP-17.

The method may further comprise the steps of saccharifying the liquefied starch to obtain dextrins; and recovering the dextrins. The method may further comprise the step of fermenting the dextrins under suitable fermentation conditions to obtain end-products. Such end-products may include alcohol (e.g. ethanol), organic acids, sugar alcohols, ascorbic acid intermediates, amino acids, and proteins.

### BRIEF DESCRIPTION OF THE DRAWINGS

Fig. 1 illustrates schematically an embodiment of the invention which includes a pretreatment step (primary liquefaction).
Fig. 2 illustrates the decrease in viscosity of a 36% ds slurry of whole ground corn exposed to phytase and alpha amylase at 85°C, pH 5.8 and reference is made to example 8.
Fig. 3 illustrates the decrease in viscosity of a 30% ds com flour slurry at pH 5.8 with a combination of SPEZYME XTRA and BP-17 as further described in example 9.
Fig. 4 illustrates the pTREX4/phytase fusion construct that was used for heterologous expression of both the wildtype and the BP-17 variant *Buttiauxella* phytase.
Fig. 5 illustrates the pTREX4/phytase direct construct that was used for heterologous expression of both the wildtype and the BP-17 variant *Buttiauxella* phytase.

### DETAILED DESCRIPTION OF THE INVENTION

### Definitions

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), arid Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

As used herein, the term "phytase" refers to an enzyme which is capable of catalyzing the hydrolysis of esters of phosphoric acid, including phytate and releasing inorganic phosphate and inositol. In some embodiments, in addition to phytate, the phytase may be capable of hydrolyzing at least one of the inositol-phosphates of intermediate degrees of phosphorylation.

The term "wild-type" as used herein refers to an enzyme naturally occurring (native) in a host cell. In some embodiments, the term "parent" or "parent sequence" is used interchangeably with the term wild-type.

The term "wild-type *Buttiauxella* phytase (WT-BP)" refers to an enzyme having the amino acid sequence of SEQ ID NO:1.

The term "*Buttiauxella* phytase -11 (BP-11)" refers to a variant phytase enzyme having the amino acid sequence of SEQ ID NO:2.

The term "*Buttiauxella* phytase -17 (BP-17)" refers to a variant phytase enzyme having the amino acid sequence of SEQ ID NO:3.

"Alpha amylases" are a -1,4-glucan-4-glucanohydrolases (E.C. 3.2.1.1) and are enzymes that cleave or hydrolyze internal α -1,4 -glycosidic linkages in starch (*e.g.* amylopectin or amylose polymers).

The term "functional equivalent" means that an enzyme has the same enzymatic functional characteristics of the *Buttiauxella* sp. phytase (WT-BP) and is derived from a wild-type phytase.

The term "variant" when used in reference to an enzyme *(e.g.* an alpha amylase, a phytase or the like) means an enzyme derived from a naturally occurring enzyme (wild-type) but having a substitution, insertion or deletion of one or more amino acids as compared to the naturally occurring enzyme. The term includes hybrid forms of the enzyme, wherein for example the enzyme may have a C-terminus derived from one *Bacillus sp.* (*e.g*., *B. licheniformis*) and an N- terminus derived from a different *Bacillus sp.* (*e.g., B. stearothermophilus*). A variant may have one or more altered properties compared to the wild-type such as but not limited to increased thermal stability, increased proteolytic stability, increased specific activity, broader substrate specificity, broader activity over a pH range or combinations thereof.

The term "contacting" refers to the placing of at least one enzyme in sufficiently close proximity to its respective substrate to enable the enzyme(s) to convert the substrate to at least one end-product. Those skilled in the art will recognize that mixing at least one solution comprising at least one enzyme with the respective enzyme substrate(s) results in "contacting."

"Liquefaction" or "liquefy" means a process by which starch is converted to shorter chain and less viscous dextrins.

"Dextrins" are short chain polymers of glucose (e.g., 2 to 10 units).

As used herein the term "starch" refers to any material comprised of the complex polysaccharide carbohydrates of plants, comprised of amylose and amylopectin with the formula (C₆H₁₀O₅)ₓ, wherein x can be any number.

The term "granular starch" means raw starch, that is, starch which has not been subject to temperatures of gelatinization.

The terms "saccharifying enzyme" and "glucoamylase (E.C. 3.2.1.3)" are used interchangeably herein and refer to any enzyme that is capable of catalyzing the release of D-glucose from the non-reducing ends of starch and related oligo-and polysaccharides.

The term "oligosaccharides" refers to any compound having 2 to 10 monosaccharide units joined in glycosidic linkages. These short chain polymers of simple sugars include dextrins.

The term "DE" or "dextrose equivalent" is an industry standard for measuring the concentration of total reducing sugars, calculated as D-glucose on a dry weight basis. Unhydrolyzed granular starch has a DE that is essentially 0 and D-glucose has a DE of 100.

The term "glucose syrup" refers to an aqueous composition containing glucose solids. Glucose syrup will have a DE of at least 20. In some embodiments, glucose syrup will not contain more than 21% water and will not contain less than 25% reducing sugar calculated as dextrose. In one embodiment, glucose syrup will include at least 90% D-glucose and in another embodiment glucose syrup will include at least 95% D-glucose. In some embodiments the terms glucose and glucose syrup are used interchangeably.

The term "total sugar content" refers to the total sugar content present in a starch composition.

The term "dry solids (ds)" refers to the total solids of a slurry in % on a dry weight basis.

As used herein, "percent (%) sequence identity" with respect to the amino acid or nucleotides sequences identified herein is defined as the percentage of amino acid residues or nucleotides in a candidate sequence that are identical with the amino acid residues or nucleotides in a sequence, after aligning the sequences and introducing gaps, if necessary, to achieve the maximum percent sequence identity, and not considering any conservative substitutions as part of the sequence identity. Methods for performing sequence alignment and determining sequence identity are known to the skilled artisan, may be performed without undue experimentation, and calculations of identity values may be obtained with definiteness. See, for example, Ausubel, et al., eds. (1995) Current Protocols in Molecular Biology, Chapter 19 (Greene Publishing and Wiley-Interscience, New York); and the ALIGN program (Dayhoff (1978) in Atlas of Protein Sequence and Structure 5:Suppl. 3 (National Biomedical Research Foundation, Washington, D.C.). A number of algorithms are available for aligning sequences and determining sequence identity and include, for example, the homology alignment algorithm of Needleman, et al., (1970) J. Mol. Biol. 48:443; the local homology algorithm of Smith, et al., (1981) Adv. Appl. Math. 2:482; the search for similarity method of Pearson et al. (1988) Proc. Natl. Acad. Sci. 85:2444; the Smith-Waterman algorithm (Meth. Mol. Biol. 70:173-187 (1997); and BLASTP, BLASTN, and BLASTX algorithms (see, Altschul, et al., (1990) J. Mol. Biol. 215:403-410). Computerized programs using these algorithms are also available, and include, but are not limited to: ALIGN or Megalign (DNASTAR) software, or WU-BLAST-2 (Altschul, et al., Meth. Enzym., 266:460-480 (1996)); or GAP, BESTFIT, BLAST Altschul, *et al.,* supra, FASTA, and TFASTA, available in the Genetics Computing Group (GCG) package, Version 8, Madison, Wis., USA; and CLUSTAL in the PC/Gene program by Intelligenetics, Mountain View, Calif. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can be determined by the Smith-Waterman homology search algorithm (Meth. Mol. Biol. 70:173-187 (1997)) as implemented in MSPRCH program (Oxford Molecular) using an affine gap search with the following search parameters: gap open penalty of 12, and gap extension penalty of 1. Preferably, paired amino acid comparisons can be carried out using the GAP program of the GCG sequence analysis software package of Genetics Computer Group, Inc., Madison, Wis., employing the blosum62 amino acid substitution matrix, with a gap weight of 12 and a length weight of 2. With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues and may be 30, 40, 50 or more amino acid residues. Corrections for increased sequence identity associated with inclusion of gaps in the derivative's amino acid sequence can be made by assigning gap penalties.

The term "% homology" is used interchangeably herein with the term "% identity". Exemplary computer programs which can be used to determine identity between two sequences include, but are not limited to, the suite of BLAST programs, *e.g*., BLASTN, BLASTX, and TBLASTX, BLASTP and TBLASTN, and are publicly available on the Internet (see, for example, the BLAST page on the National Center for Biotechnology Information website). See also, Altschul, *et al.,* 1990 and Altschul, *et al.,* 1997.

Sequence searches are typically carried out using the BLASTN program when evaluating a given nucleic acid sequence relative to nucleic acid sequences in the GenBank DNA Sequences and other public databases. The BLASTX program is preferred for searching nucleic acid sequences that have been translated in all reading frames against amino acid sequences in the GenBank Protein Sequences and other public databases. Both BLASTN and BLASTX are run using default parameters of an open gap penalty of 11.0, and an extended gap penalty of 1.0, and utilize the BLOSUM-62 matrix. (See, *e.g.,* Altschul, *et al.,* 1997.)

A preferred alignment of selected sequences in order to determine "% identity" between two or more sequences, is performed using for example, the CLUSTAL-W program in Mac Vector version 6.5, operated with default parameters, including an open gap penalty of 10.0, an extended gap penalty of 0.1, and a BLOSUM 30 similarity matrix.

The term "milled" is used herein to refer to plant material that has been reduced in size, such as by grinding, crushing, fractionating or any other means of particle size reduction. Milling includes dry or wet milling. "Dry milling" refers to the milling of whole dry grain. "Wet milling" refers to a process whereby grain is first soaked (steeped) in water to soften the grain.

The term "gelatinization" means solubilization of a starch molecule, generally by cooking, to form a viscous suspension.

The term "gelatinization temperature" refers to the lowest temperature at which gelatinization of a starch containing substrate begins. The exact temperature of gelatinization depends on the specific starch and may vary depending on factors such as plant species and environmental and growth conditions.

The term "below the gelatinization temperature" refers to a temperature that is less than the gelatinization temperature.

The term "slurry" refers to an aqueous mixture comprising insoluble solids, (e.g. granular starch).

The term "fermentation" refers to the enzymatic and anaerobic breakdown of organic substances by microorganisms to produce simpler organic compounds. While fermentation occurs under anaerobic conditions it is not intended that the term be solely limited to strict anaerobic conditions, as fermentation also occurs in the presence of oxygen.

The phrase "simultaneous saccharification and fermentation (SSF)" refers to a process in the production of end products in which a fermenting organism, such as an ethanol producing microorganism, and at least one enzyme, such as a saccharifying enzyme are combined in the same process step in the same vessel.

The term "thin stillage" means the liquid portion of stillage separated from the solids (e.g., by screening or centrifugation) which contains suspended fine particles and dissolved material. The term "backset" and/or "make-up water" is generally used to mean recycled thin stillage.

The term "end product" refers to any carbon-source derived product which is enzymatically converted from a fermentable substrate. In some preferred embodiments, the end product is an alcohol (e.g., ethanol).

The term "derived" encompasses the terms "originated from", "obtained" or "obtainable from", and "isolated from" and in some embodiments as used herein means that a polypeptide encoded by the nucleotide sequence is produced from a cell in which the nucleotide is naturally present or in which the nucleotide has been inserted.

As used herein the term "fermenting organism" refers to any microorganism or cell, which is suitable for use in fermentation for directly or indirectly producing an end product.

As used herein the term "ethanol producer" or ethanol producing microorganism" refers to a fermenting organism that is capable of producing ethanol from a mono- or oligosaccharide.

The terms "recovered", "isolated", and "separated" as used herein refer to a protein, cell, nucleic acid or amino acid that is removed from at least one component with which it is naturally associated.

The terms "protein" and "polypeptide" are used interchangeably herein. In the present disclosure and claims, the conventional one-letter and three-letter codes for amino acid residues are used. The 3-letter code for amino acids as defined in conformity with the IUPAC-IUB Joint Commission on Biochemical Nomenclature (JCBN). It is also understood that a polypeptide may be coded for by more than one nucleotide sequence due to the degeneracy of the genetic code.

The term "operably linked" refers to juxtaposition wherein the elements are in an arrangement allowing them to be functionally related. For example, a promoter is operably linked to a coding sequence if it controls the transcription of the sequence.

The term "selective marker" refers to a gene capable of expression in a host that allows for ease of selection of those hosts containing an introduced nucleic acid or vector. Examples of selectable markers include but are not limited to antimicrobials (e.g., hygromycin, bleomycin, or chloramphenicol) and/or genes that confer a metabolic advantage, such as a nutritional advantage on the host cell.

The term "heterologous" with reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes.

The term "endogenous" with reference to a polynucleotide or protein refers to a polynucleotide or protein that occurs naturally in the host cell.

As used herein, the terms "transformed", "stably transformed" and "transgenic" used in reference to a cell means the cell has a non-native (e.g., heterologous) nucleic acid sequence integrated into its genome or as an episomal plasmid that is maintained through multiple generations.

As used herein, the term "expression" refers to the process by which a polypeptide is produced based on the nucleic acid sequence of a gene. The process includes both transcription and translation.

The term "introduced" in the context of inserting a nucleic acid sequence into a cell, means "transfection", or "transformation" or "transduction" and includes reference to the incorporation of a nucleic acid sequence into a eukaryotic or prokaryotic cell wherein the nucleic acid sequence may be incorporated into the genome of the cell (*e.g*., chromosome, plasmid, plastid, or mitochondrial DNA), converted into an autonomous replicon, or transiently expressed (*e.g.*, transfected mRNA).

It must be noted that as used herein and in the appended claims, the singular forms "a", "an", and "the" include plural referents unless the context clearly dictates otherwise. Thus, for example, reference to "a cell" includes reference to one or more cells and equivalents thereof known to those skilled in the art, and so forth.

Where a range of values is provided, it is understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Other definitions of terms may appear throughout the specification.
Before the exemplary embodiments are described in more detail, it is to be understood that this invention is not limited to particular embodiments described and, as such, may, of course, vary.

### Exemplary embodiments

The present inventors have found addition of a phytase, specifically a wild-type *Buttiauxella sp.* phytase (*e.g.* P1-29, SEQ ID NO:1) and variants thereof (*e.g.*, BP-11, SEQ ID NO:2 and BP-17, SEQ ID NO:3), to a starch hydrolysis process comprising an alpha amylase provides for certain advantages over the use of the alpha amylase without the phytase. In addition, the inventors have found that the use of a wild-type *Buttiauxella sp.* phytase (*e.g.* P 1-29) and variants thereof provides means of increasing the efficiency of starch hydrolysis.

Current ethanol processes require pH adjustments before and after starch liquefaction to provide the appropriate conditions for liquefaction enzymes and yeast fermentation. The pH adjustment results in a number of disadvantages. For example, adjusting the pH causes high salt which can inhibit the fermentation organisms. If sulfuric acid is used, it can also result in sulfur disposal problems. In addition, pH adjustment requires additional steps in the process, reducing efficiency. By stabilizing liquefaction enzymes (e.g., alpha amylases) with a phytase, it was found that the liquefaction could occur at a lower pH than it would normally require. In fact, the treatment with phytases could result in liquefaction occurring at the pH of the whole ground grain slurry without pH adjustment, even whole ground slurry containing high levels of thin stillage. This allowed for the elimination of the pH adjustment using alkali or acids during the conversion of whole ground grain to ethanol. This further enabled the conversion of starch to glucose in a single liquefaction step without pH adjustment or in two liquefaction steps utilizing low dosages of the enzyme. As an added advantage, this enabled the process to proceed to simultaneous saccharification and fermentation without any further pH adjustment. In addition, even if the process is allowed to proceed with pH adjustments, it resulted in increased thermostability of the alpha amylase.

### Phytases -

In some embodiments, the at least one phytase useful in the present invention is one derived from the bacterium *Buttiauxella spp.* The *Buttiauxella spp.* includes *B. agrestis, B. brennerae, B. ferragutiase, B. gaviniae, B. izardii, B. noackiae,* and *B. warmboldiae.* Strains of *Buttiauxella* species are available from DSMZ, the German National Resource Center for Biological Material (Inhoffenstrabe 7B, 38124 Braunschweig, Germany). *Buttiauxella sp.* strain P1-29 deposited under accession number NCIMB 41248 is an example of a particularly useful strain from which a phytase may be obtained and used according to the invention. Phytases may be identified from *Buttiauxella spp.* by methods described in WO 06/ 043178, for example by hybridization techniques.

In a preferred embodiment, a phytase useful in the instant invention is one having at least 90%, at least 93%, at least 95%, at least 96%, at least 97%, at least 98% and at least 99% sequence identity to the amino acid sequence set forth in SEQ ID NO: 1 (see Table 1). More preferably, a phytase useful in the present invention is one having at least 95% to 99% sequence identity to the amino acid sequence set forth in SEQ ID NO:1 or variants thereof. In some embodiments, the phytase comprises the amino acid sequence of SEQ ID NO:1.

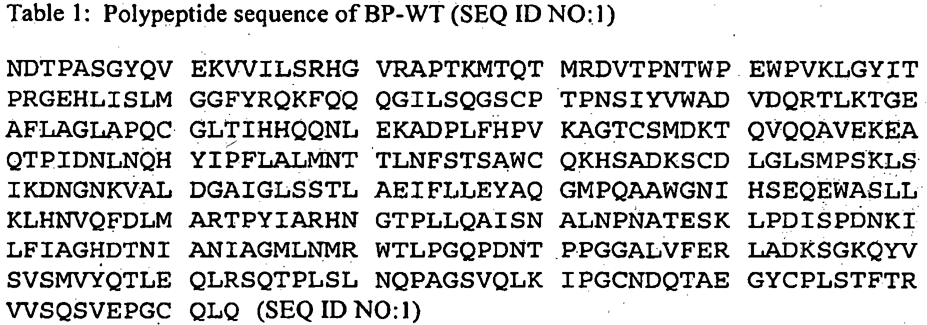

In other some embodiments, the phytase is a variant of the phytase having an amino acid sequence as set forth in SEQ ID NO:1. Some preferred variants are the variants disclosed in PCT patent publication WO 2006/043178.

Other preferred variants of SEQ ID NO: 1 include polypeptides comprising a mutation in at least one of the following positions of SEQ ID NO:1 of the instant disclosure: 26, 37, 89, 92, 134, 160, 164, 171, 176, 178, 188, 190, 192, 207, 209, 211, 235, 248, 256, 261, 270, 306 or 318. In some embodiments, the variant will include phytase polypeptides comprising a mutation in the following positions corresponding to SEQ ID NO:1: 89, 134, 164, 176, 178, 207, 209, 248, 256,261 and 270. In other embodiments, the variant will include at least 1 further mutation in a position corresponding to SEQ ID NO:1.

In other embodiments, the phytase will comprise at least one mutation in a position corresponding to K26E, T134V/I, F164S, T176K, K207T/E, D211C, or Q256Y. In other embodiments, the variant includes polypeptides comprising a combination of mutations. For example, reference is made to Table 1 of WO 2006/ 043178, wherein the numbering is in reference to SEQ ID NO:3 of the published PCT application. In some embodiments, the variant has at least 40%, 50%, 60%, 70%, 80%, 90%, 95%, 98%, 99%, or 100% of the phytase activity of the parent or wildtype phytase of SEQ ID NO:1.

In some embodiments of the instant invention, the phytase comprises or consists of the amino acid sequence of SEQ ID NO:2 or phytases having at least 95%, at least 96%, at least 97%, at least 98% and at least 99% amino acid sequence identity thereto. The phytase comprising or consisting of the amino acid sequence of SEQ ID NO:2 herein is also disclosed in WO 2006/043178 as a variant of the wild-type *Buttiauxella spp.* This variant is referred to herein as BP-11. There are 11 amino acid residues which are different between the amino acid sequence of SEQ ID NO:1 (BP-WT) and the BP-11 variant (SEQ ID NO:2), and these residues are in bold and underlined in Table 2 in the amino acid sequence of SEQ ID NO:2. In some embodiments, the phytase will include the BP-11 phytase, wherein there may be 1, 2, 3 or more amino acid changes.

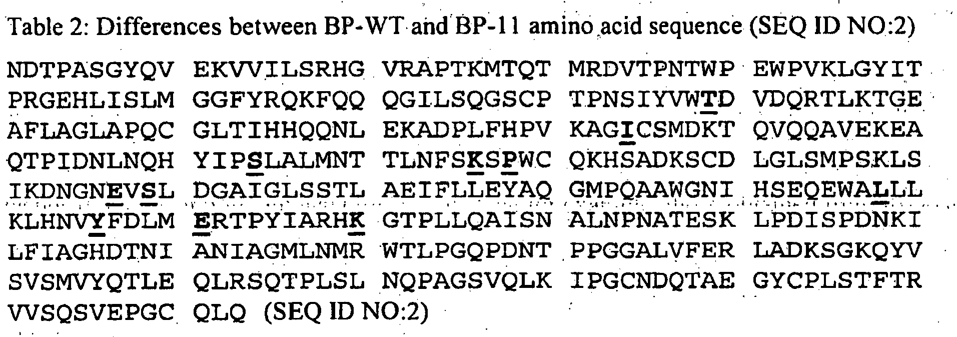

In some embodiments, of the instant invention, the variant will comprise or consist of a substitution in amino acid residues corresponding to residue positions A89, D92, T134, F174, T186, A188, K207, A209, S248, Q256, A261, and N269 of SEQ ID NO:1. In some embodiments, the phytase comprises or consists of the amino acid sequence of SEQ ID NO:3 or phytases having at least 95%, at least 96%, at least 97%, at least 98% and at least 99% amino acid sequence identity thereto. The variant of SEQ ID NO:3 is referred to herein as BP-17. There are 12 amino acid residues which are different between the amino acid sequence of SEQ ID NO: 1 and the BP-17 variant (SEQ ID NO:3) and reference is made to the following substitutions A89T, D92A, T134I, F174S, T186K, A188P, K207E, A209S, S248L, Q256Y, A261E, and N269K. In addition BP-17 differs from BP-11 in one amino acid substitution at the position corresponding to residue 92. Thus, in some embodiments, the variant has at least 90%, 95%, 96%, 97%, 98%, and 99% sequence identity to SEQ ID NO:1 and has at least an Alanine at amino acid 92. In other embodiments, the variant has at least 95%, 96%, 97%, 98%, and 99% sequence identity to SEQ ID NO:3 and has at least an Alanine at amino acid 92. In other embodiments, the variant has at least 95%, 96%, 97%, 98%, and 99% sequence identity to SEQ ID NO:1 and has at least a Alanine at amino acid 92 and has at least one other amino acid substitution selected from the group of: A89T, T134I, F174S, T186K, A188P, K207E, A209S, S248L, Q256Y, A261E, and N269K. The difference between the residues of BP-WT and BP-17 are in bold and underlined in Table 3 below:

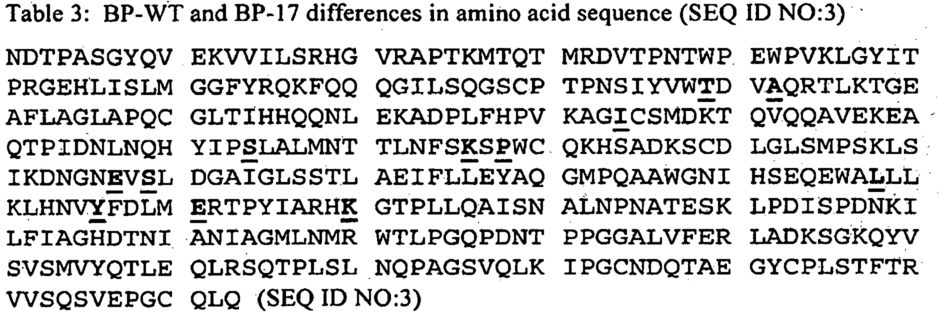

In some embodiments, the phytase having at least 95% sequence identity to SEQ ID NO:2 or SEQ ID NO:3 will include a conservative amino acid replacement. Conservative amino acid replacements include for example, for Ala replacement with Gly or Cys; for Arg replacement with Lys, Met or Ile; for Asn replacement with Asp or Glu; for Asp replacement with Asn or Gln; for Cys replacement with Met or Thr; for Gln replacement with Asn, Glu, or Asp; for Gly replacement with Ala or Pro; for Ile replacement with Val, Leu, or Met; for Leu replacement with Val or Met; for Lys replacement with Arg, Met or Ile; for Met replacement with Cys, Ile, Leu or Val; for Phe replacement with Tyr, His, Trp; for Ser replacement with Thr, Met or Cys; for Thr replacement with Ser, Met or Val; for Tyr replacement with Phe, or His and for Val replacement with Leu, Ile or Met.

### Alpha amylases -

In some embodiments, the alpha amylase is an acid stable alpha amylase which when added in an effective amount has activity in the pH range of 3.0 to 7.0 and preferably from 3.5 to 6.5. Alpha amylases useful according to the invention may be fungal alpha amylases or bacterial alpha amylases. Further the alpha amylase may be a wild-type alpha amylase, a variant or fragment thereof or a hybrid alpha amylase which is derived from, for example, a catalytic domain from one microbial source and a starch binding domain from another microbial source.

Some examples of fungal alpha amylases include those obtained from filamentous fungal strains including but not limited to strains of *Aspergillus* (*e.g., A. niger, A. kawachi,* and *A*. *oryzae*); *Trichoderma sp., Rhizopus sp., Mucor sp.,* and *Penicillium sp.*

More preferably, the acid stable alpha amylase is derived from a bacterial strain. Bacterial strains include without limitation *Bacillus sp., Streptomyces sp.* and *Lactobacillus sp.* Some bacterial strains include *Bacillus sp.,* such as *B. licheniformis, B. stearothermophilus, B. amyloliquefaciens, B. subtilis, B. lentus,* and *B. coagulans.* Particualrly, *B. licheniformis, B. stearothermophilus* and *B. amyloliquefaciens.*

Preferably one of the bacterial alpha amylases used in the compositions and processes of the invention include one of the alpha amylases described in USP 5,093,257; USP 5,763,385; USP 5,824,532; USP 5,958,739; USP 6,008,026; USP 6,093,563; USP 6,187,576; USP 6,297,038; USP 6,361,809; USP 6,867,031; US 2006/0014265; WO 96/23874, WO 96/39528; WO 97/141213, WO 99/19467; and WO 05/001064.

Commercially available alpha amylases contemplated for use in the compositions and method encompassed by the invention include: SPEZYME^{®} AA; SPEZYME^{®} FRED; SPEZYME^{®} XTRA; GZYME 997; and CLARASE L (Genencor International Inc.); TERMAMYL 120-L, LC and SC and SUPRA (Novozymes Biotech); LIQUOZYME X and SAN SUPER (Novozymes A/S) and ULTRA THIN (Diversa/Valley Research). In some embodiments, SPEZYME XTRA and/or SPEZYME FRED as described in WO 05/111203, are used in combination with a *Buttiauxella* phytase or variant.

In some embodiments, the alpha amylase is a Termamyl-like alpha amylase. Termamyl-like alpha amylases are intended to indicate alpha amylases which at the amino acid level exhibit a substantial homology to the *B. licheniformis* alpha amylase, such as having at least 75% sequence identity, including 80%, 85%, 90%, 95%, 99% and 100% sequence identity with the B. *licheniformis* alpha amylase designated as SEQ ID NO:4 in WO 06/066594.

The enzyme compositions employed by the invention may include blended or formulated enzyme compositions. The enzyme components can be used as a blended formulation comprising at least the two enzyme components mixed together (a mixture) or the enzyme components can be individually added during one or more process steps. This may involve adding the separate enzyme components in a time-wise manner such that an enzyme ratio of phytase to amylase is maintained, for example adding the components simultaneously. By formulated enzyme compositions means that the enzymes are provided individually in a formulated manner, such as a specific ratio. In some embodiments, the compositions will include a phytase having at least 90% sequence identity with SEQ ID NO:1; and/or a phytase having at least 95% sequence identity with SEQ ID NO:2; and/or a phytase having at least 95% sequence identity with SEQ ID NO:3 and an alpha amylase.

In some embodiments, the alpha amylase will include an alpha amylase derived from *Bacillus sterarothermophilus* such as SPEZYME AA, LIQUOZYME or SPEZYME XTRA. In some embodiments, the alpha amylase will include an alpha amylase derived from *Bacillus licheniformis.* In some embodiments, the alpha amylase will be a hybrid enzyme for example the hybrid enzyme may comprise fragments that are derived from a strain of *B. stearothermophilus* and a strain of *B. licheniformis.*

In some embodiments, the enzyme compositions or blend will include: a) BP-WT, SPEZYME XTRA and optionally SPEZYME FRED; b) variants of BP-WT, SPEZYME XTRA and optionally SPEZYME FRED; c) BP-17, SPEZYME XTRA and optionally SPEZYME FRED. In some embodiments, the composition includes an alpha amylase and a *Buttiauxella* phytase. In some embodiments, the composition includes SPEZYME™ XTRA and BP-WT. In some embodiments, the composition includes SPEZYME™ XTRA and BP-17. In Some embodiments, the composition includes SPEZYME™ XTRA and a phytase having at least 75% sequence identity to SEQ ID NO:1, SEQ ID NO:2 and/or SEQ ID NO:3, including 80%, 85%, 90%, 95%, and 99% sequence identity. In some embodiments, the composition includes SPEZYME™ FRED and BP-WT. In some embodiments, the composition includes SPEZYME™ FRED and BP-17. In Some embodiments, the composition includes SPEZYME™ FRED and a phytase having at least 75% sequence identity to SEQ ID NO:1, SEQ ID NO:2 and/or SEQ ID NO:3, including 80%, 85%, 90%, 95%, and 99% sequence identity.

Enzyme compositions comprising the phytase and alpha amylase either in a blended formulation or individually include starch hydrolysis compositions, for example, MAXALIQ™ One from Danisco US, Inc., Genencor Division. In certain embodiments, the phytase may be combined with an alpha amylase such as LIQUOZYME, TERMAMYL LC or SUPRA. Mixtures of alpha amylases for use in starch liquefaction are known and reference is made to USP 4,933,279 which discloses a mixed enzyme product comprising a mixture of an alpha amylase from *B. licheniformis* and an alpha amylase *from B. stearothermophilus.*

In some embodiments, the inclusion of the phytase and an alpha amylase, whether the enzymes are provided in a blend or individually, allows for the process of starch liquefaction at a pH lower than what could be used if the alpha amylase was provided without said phytase. For example, the starch liquefaction process can be conducted at a pH of about 0.5 to about 1.5 units lower (*e.g*. about 0.2, 0.3, 0.4, 0.5, 0.7, 0.8, 1.0., 1.2 or 1.5 units lower) than if the alpha amylase was used without the phytase encompassed by the invention.

In some embodiments, when a phytase composition and an alpha amylase composition are used, for example, in a process for starch hydrolysis, the ratio of phytase (FTU/g ds) to alpha amylase (AAU/g ds) is from about 15:1 to about 1:15, alternatively about 10:1 to about 1:10, alternatively about 5:1 to about 1:5, alternatively about 3:1 to about 1:2, including about 1:1, about 1.1:1, about 1.2:1, about 1.3:1, about 1.4:1, about 1.5:1, about 1.6:1, about 1.7:1, about 1.8:1, about 1.9:1, and about 2:1.

Some useful enzyme compositions comprising the phytase and alpha amylase either in a blended formulation or individually include starch hydrolysis compositions which are discussed in further detail under the heading "Methods."

### Secondary Enzymes -

While some embodiments of the invention include a composition or a blend of an alpha-amylase and a phytase, the composition can optionally include other enzymes. Alternatively, other enzymes can be added separately from the composition at various points during the methods of the invention at the same time as the composition or at a different time in the process. For example, other components useful during liquefaction include without limitation: cellulases, hemicellulases, xylanases, proteases, phytases, pullulanases, beta amylases, lipases, cutinases, pectinases, beta-glucanases, beta-glucosidases, galactosidases, esterases, cyclodextrin transglycosyltransferases (CGTases), beta-amylases and combinations thereof.

Glucoamylases (E.C. 3.2.1.3.) can be derived from the heterologous or endogenous protein expression of bacterial, plant and/or fungal sources. Some glucoamylases useful in the invention are produced by several strains of filamentous fungi and yeast. In particular, glucoamylases secreted from strains of *Aspergillus* and *Trichoderma* are commercially important. Suitable glucoamylases include naturally occurring wild-type glucoamylases as well as variant and genetically engineered mutant glucoamylases (e.g. hybrid glucoamylases). The following glucoamylases are non-limiting examples of glucoamylases that can be used in the process encompassed by the invention. *Aspergillus niger* G1 and G2 glucoamylase (see e.g., Boel et al., (1984) EMBO J. 3:1097 - 1102; WO 92/00381, WO 00/04136 and USP 6,352,851); *Aspergillus awamori* glucoamylases (see e.g., WO 84/02921); *Aspergillus oryzae* glucoamylases (see e.g., Hata et al., (1991) Agric. Biol. Chem. 55:941 - 949) and *Aspergillus shirousami.* (See e.g., Chen et al., (1996) Prot. Eng. 9:499 - 505; Chen et al. (1995) Prot. Eng. 8:575-582; and Chen et al., (1994) Biochem J. 302:275-281).

Glucoamylases are also obtained from strains of *Talaromyces* such as those derived from *T. emersonii, T. leycettanus, T. duponti* and *T. thermophilus* (see e.g., WO 99/28488; USP No. RE: 32,153; USP No. 4,587,215); strains of *Trichoderma,* such as *T. reesei* and particularly glucoamylases having at least about 80%, 85%, 90% and 95% sequence identity to SEQ ID NO: 4 disclosed in US Pat. Pub. No. 2006-0094080; strains of *Rhizopus,* such as *R. niveus and R. oryzae;* strains of *Mucor* and strains of *Humicola*, such as *H. grisea* (See, e.g., Boel et al., (1984) EMBO J. 3:1097-1102; WO 92/00381; WO 00/04136; Chen et al., (1996 Prot. Eng. 9:499-505; Taylor et al., (1978) Carbohydrate Res. 61:301-308; USP. 4,514,496; USP 4,092,434; USP 4,618,579; Jensen et al., (1988) Can. J. Microbiol. 34:218 - 223 and SEQ ID NO: 3 of WO 2005/052148). In some embodiments, the glucoamylase will have at least about 85%, 90%, 92%, 94%, 95%, 96%, 97%, 98% and 99% sequence identity to the amino acid sequence of SEQ ID NO: 3 of WO 05/052148. Other glucoamylases useful in the present invention include those obtained from *Athelia rolfsii* and variants thereof (see e.g., WO 04/111218). See also the discussion about saccharification herein for examples of other glucoamylases.

Enzymes having glucoamylase activity used commercially are produced, for example, from *Aspergillus niger* (see e.g., trade name DISTILLASE, OPTIDEX L-400 and G ZYME G990 4X from Danisco US, Inc, Genencor Division.) or *Rhizopus* species (see e.g., trade name CU CONC from Shin Nihon Chemicals, Japan). Also the commercial digestive enzyme, trade name GLUCZYME from Amano Pharmaceuticals, Japan (see e.g., Takahashi et al, (1985) J. Biochem. 98:663-671). Additional enzymes include three forms of glucoamylase (E.C.3.2.1.3) of a Rhizopus sp., namely "Gluc1" (MW 74,000), "Gluc2" (MW 58,600) and "Gluc3" (MW 61,400). Also the enzyme preparation GC480 (Danisco US, Inc, Genencor Division) finds use in the invention. The above mentioned glucoamylases and commercial enzymes are not intended to limit the invention but are provided as examples only.

In some embodiments the additional enzyme is a second alpha amylase such as a bacterial or fungal alpha amylase, and in other embodiments the alpha amylase is a derivative, mutant or variant of a fungal or bacterial alpha amylase. Any alpha amylases can be used, including those known in the art as well as those discussed herein. Non-limiting examples of alpha amylases useful in combination with at least one alpha amylase and phytase according to the invention are those derived from *Bacillus, Aspergillus, Trichoderma, Rhizopus, Fusarium*, *Penicillium, Neurospora* and *Humicola.*

Some additional alpha amylases are derived from *Bacillus* including *B. licheniformis, B. lentus, B. coagulans, B. amyloliquefaciens, B. stearothermophilus, B sublilis,* and hybrids, mutants and variants thereof (see e.g., USP 5,763,385; USP 5,824,532; USP 5,958,739; USP 6,008,026 and USP 6,361,809). Some of these amylases are commercially available e.g., TERMAMYL and SUPRA available from Novo Nordisk A/S, ULTRATHIN from Diversa, LIQUEZYME SC from Novo Nordisk A/S and SPEZYME FRED, SPEZYME XTRA and GZYME G997 available from Danisco US, Inc, Genencor Division.

In another embodiment, the invention will include the addition of a second phytase. Any of the phytases discussed in the section herein on phytases can be used.

Cellulases can also be incorporated with the alpha amylase and glucoamylase. Cellulases are enzyme compositions that hydrolyze cellulose (β-1, 4-D-glucan linkages) and/or derivatives thereof, such as phosphoric acid swollen cellulose. Cellulases include the classification of exo-cellobiohydrolases (CBH), endoglucanases (EG) and β-glucosidases (BG) (EC3.2.191, EC3.2.1.4 and EC3.2.1.21). Examples of cellulases include cellulases from *Penicillium, Trichoderma, Humicola, Fusarium, Thermomonospora, Cellulomonas, Clostridium* and *Aspergillus.* Commercially available cellulases sold for feed applications are beta-glucanases such as ROVABIO (Adisseo), NATUGRAIN (BASF), MULTIFECT BGL (Danisco US, Inc, Genencor Division) and ECONASE (AB Enzymes).

Xylanases can also be included. Xylanases (e.g. endo-β-xylanases (E.C. 3.2.1.8), which hydrolyze the xylan backbone chain may be from bacterial sources, such as *Bacillus, Streptomyces, Clostridium, Acidothermus, Microtetrapsora or Thermonospora.* In addition xylanases may be from fungal sources, such as *Aspergillus, Trichoderma, Neurospora, Humicola, Penicillium* or *Fusarium*. (See, e.g., EP473 545; USP 5,612,055; WO 92/06209; and WO 97/20920). Commercial preparations include MULTIFECT and FEEDTREAT Y5 (Danisco US, Inc, Genencor Division), RONOZYME WX (Novozymes A/S) and NATUGRAIN WHEAT (BASF).

Proteases can also be included. Proteases can be derived from *Bacillus* such as *B. amyloliquefaciens, B. lentus, B. licheniformis,* and *B. subtilis.* These sources include subtilisin such as a subtilisin obtainable from *B. amyloliquefaciens* and mutants thereof (USP 4,760,025). Suitable commercial protease includes MULTIFECT P 3000 (Danisco US, Inc, Genencor Division) and SUMIZYME FP (Shin Nihon). Proteases are also derived from fungal sources such as *Trichoderma* (e.g., NSP-24), *Aspergillus, Humicola* and *Penicillium.*

In some embodiments, combinations of two or more enzymes selected from alpha amylases, glucoamylases, phytases, cellulases, hemicellulases, and xylanases can be included.

Acid fungal proteases (AFP) can also be included in the enzyme compositions and blends of the invention. Fungal proteases include for example, those obtained from *Aspergillus*, *Trichoderma, Mucor* and *Rhizopus*, such as *A. niger, A. awamori, A. oryzae* and *M. miehei* (see e.g. US application number 11/312,290, filed December 20, 2005, for an AFP useful in the invention).

### Methods of Use -

The methods of the invention involve the use of a phytase of the invention with an alpha amylase during liquefaction of starch in starch conversion processes, resulting in a process that can occur at a lower pH. In some embodiments, the method no longer requires the addition of acids or alkali for pH adjustment. The methods include a primary liquefaction step and a secondary liquefaction step. In some embodiments, the method results in production of fermentable sugars. In some embodiments, the process results in ethanol.

Substrates useful according to the invention include grains and/or plant material comprising granular starch. Plant material may be obtained from plants including but not limited to wheat, corn, rye, sorghum (milo), rice, millet, barley, triticale, cassava (tapioca), potato, sweet potato, sugar beets, sugarcane, and legumes such as soybean and peas. Plant material usable in the invention includes corn, barley, wheat, rice, milo and combinations thereof. Plant material may include hybrid varieties and genetically modified varieties (*e.g*. transgenic corn, barley or soybeans comprising heterologous genes). Any part of the plant may be used to provide substrate including but not limited to plant parts such as leaves, stems, hulls, husks, tubers, cobs, grains and the like. In some embodiments, essentially the entire plant may be used, for example, the entire corn stover may be used. In some embodiments, whole grain may be used as a source of granular starch. Whole grains include corn, wheat, rye, barley, sorghum and combinations thereof. In other embodiments, granular starch may be obtained from fractionated cereal grains including fiber, endosperm and/or germ components. Methods for fractionating plant material such as corn and wheat are known in the art. In some embodiments, plant material obtained from different sources may be mixed together to obtain substrates used in the processes of the invention (*e.g*. corn and milo or corn and barley).

In some embodiments, plant material may be prepared by means such as milling. Two general milling processes are preferred for the methods of the invention and these include wet milling or dry milling. In dry milling for example, the whole grain is milled and used in the process. In wet milling the grain is separated (e.g. the germ from the meal). In particular, means of milling whole cereal grains are well known and include the use of hammer mills and roller mills. Methods of milling are well known in the art and reference is made TO THE ALCOHOL TEXTBOOK: A REFERENCE FOR THE BEVERAGE, FUEL AND INDUSTRIAL ALCOHOL INDUSTRIES 3rd ED. K.A. Jacques et al., Eds, (1999) Nottingham University Press. See, Chapters 2 and 4.

In some embodiments, the plant material whether reduced by milling or other means will be combined with a solution resulting in a slurry comprising starch substrate. In some embodiments, the slurry may include a side stream from starch processing such as backset. In some embodiments, the slurry will comprise 15 - 55% ds (e.g., 20 - 50%, 25 - 45%, 25 - 40%) and 20 - 35% ds).

In some embodiments, a combination of the phytase and alpha amylase can be added to a slurry of milled starch substrate (*e.g.* milled grain). The slurry is maintained at a pH range of about 4.0 to about 6.2, also at a pH range of about 4.5 to less than about 6.0 and preferably at a pH range of about 5.0 to about 6.0 (*e.g.* about 5.4 to about 5.8), and the milled granular starch in the slurry can be contacted with the enzyme composition for a period of 2 mins to 8 hrs (e.g. 5 min to 6 hrs, 5 min to 4 hrs and 30 min to 4 hrs) to obtain liquefied starch. In some embodiments, the temperature will be in the range of 40 to 115°C. In some embodiments, the temperature will be in the range of 40 to 110°C; also 50 to 110°C; also 60 to 110°C; also 60 to 100°C and also 70 to 95°C.

In some embodiments, the phytase is added to the slurry in an amount sufficient to allow the process to occur at a lower pH, even as low as the pH of the slurry without addition of acid or base. In some embodiments, the phytase reduces phytic acid levels in an amount sufficient to increase thermostability of the alpha amylase. In some embodiments, the IP6 phytic acid is reduced. IP6 is defined as inositol containing 6 phosphate groups. IP6 is usually found with various amounts of its derivatives each having 1 to 5 phosphate groups (IPS-IP1).

In some embodiments, the phytase is added in an amount and for a time sufficient to result in increase of the thermostability of the alpha amylase. In some embodiments, the phytase is added in an amount sufficient to allow hydrolysis of starch in the presence of alpha amylase at a lower pH. In some embodiments the phytase is added in an amount sufficient to increase the thermostability of the alpha amylase at lower pH. It is to be understood that even if the pH adjustments are included in the process, the phytase results in increased thermostability of alpha amylase, particularly at a lower pH than that at which it would be stable without the phytase.

One skilled in the art will be able to readily determine the effective dosage of phytase and alpha amylase to be used in the processes according to the invention. The optimal usage level in a starch liquefaction depends upon processing parameters such as type of plant material, viscosity, processing time, pH, temperature and ds. As a general guideline, in some embodiments, the amount (dosage) of phytase used in the liquefaction process will be in the range of about 0.001 to about 50 FTU/gds, in some embodiments about 0.01 to about 5.0 FTU/gds; alternatively about 0.05 to about 10 FTU/gds, and also about 0.10 to about 5.0 FTU/gds.

In some embodiments, the amount of alpha amylase will be an effective amount of alpha amylase which is well known to a person of skill in the art. In some embodiments, the range will be about 0.05 to about 50 AAU/gds, also about 0.1 to about 20 AAU/gds and also about 1.0 to about 10 AAU/gds. In some embodiments, the range of alpha amylase will be about 0.5 to about 100 LU/gds, also about 1.0 to about 50 LU/gds, and also about 5.0 to about 25 LU/gds LU. In further embodiments, the alpha amylase dosage will be in the range of about 0.01 to about 10.0 kg/metric ton (MT)ds; also about 0.05 to about 5.0 kg/MT ds; and also about 0.1 to about 4.0 kg/MT ds.

The present invention exposes a slurry comprising a substrate such as a granular starch substrate (e.g. milled grain) to a pretreatment (incubation) step.

The incubation step includes contacting the slurry comprising a starch substrate (e.g. milled grain) with a phytases and an alpha amylase at a temperature above 55°C and below the starch gelatinization temperature of the granular starch. This temperature may be 0 to 25°C, 0 to 20°C, 0 to 15°C and 0 to 10°C below the starch gelatinization temperature. This specific value will vary and depends on the type of granular starch comprising the slurry. For example, the starch gelatinization temperature of corn is generally higher than the starch gelatinization temperature of rye or wheat. In some embodiments, the temperature will be below 68°C; below 65°C, below 62°C or below 60°C. In some embodiments, the temperature of the incubation will be between about 58 and about 72°C and also between about 60 an about 68°C. The incubation is conducted at a pH range of about 4.0 to about 6.2, e.g. about 4.0 to about 6.0, or about pH 5.0 to about 6.0 for a period of time of about 2 minutes to about 4 hours (*e.g*., about 5 mins to about 3 hrs; about 15 mins to about 2.5 hrs and about 30 min to about 2 hrs). The phytase and alpha amylase may be added as a mixture or added sequentially during the incubation step.

In a further step the incubated substrate is liquefied by exposing the incubated substrate to an increase in temperature of 0 to 45°C above the starch gelatinization temperature. (*e.g.* to 65°C to 120°C, 70°C to 110°C, 70°C to 90°C) for a period of time of 5 minutes to 6 hours (*e.g.,* 5 minutes to 4 hours and preferably 1hr to 2 hrs) at a pH of about 4.0 to about 6.2.

In some embodiments, a thermostable alpha amylase will be added to this step but in other embodiments, no additional alpha amylase will be added. In some embodiments, the pH of the incubation and the elevated temperature step will be conducted at essentially the same pH range (*e.g*., pH about 4.5 to about 6.0 or pH about 5.0 to about 6.0).

In some embodiments, the incubation step will enhance the stability of the alpha amylase during the step of temperature elevation. In some embodiments, the enhancement of alpha amylase stability is at a pH range of about 5.8 to about 5.2.

The method may further comprise saccharifying the liquefied substrate with a saccharifying enzyme (e.g., OPTIDEX L-400, OPTIMAX 4060 VHP, FERMENZYME L-400, DISTILLASE, GZYME 480 (Danisco US, Inc. Genencor Division) to obtain dextrins; and can include recovering the same. These methods are well known in the art and comprise the addition of saccharifying enzymes such as glucoamylases and optionally other secondary enzymes.

The saccharification process may last for 12 to 120 hours. However, it is common to perform a pre-saccharification for 30 minutes to 2 hours and then complete the saccharification during fermentation. Sometimes this is referred to as simultaneous saccharification and fermentation (SSF). Saccharification is commonly carried out at temperatures of 30 to 65°C and typically at pH of 4.0 to 5.0.

Glucoamylases (GA) (E.C. 3.2.1.3.) for use as saccharfying enzymes can be any of those known to the skilled artisan as well as any of those discussed herein, such as in the section heading "Secondary Enzymes."

The viscosity of the liquefact which is obtained after elevation of the temperature may have reduced viscosity as compared to a corresponding liquefact that was not incubated with a phytase encompassed by the invention. In some embodiments, due to this reduction in viscosity, the amount of alpha amylase that will be used in a starch hydrolysis process will be decreased. For example under the same conditions the dose of alpha amylase that may be needed at the same pH (e.g. about pH 5.5 to about 6.0) to obtain the same level of viscosity may be about 20%, 30%, 40%, 50%, or 60% less when the alpha amylase is combined with a phytase of the invention.

In some embodiments, the methods encompassing the use of a *Buttiauxella* phytase or variants thereof and alpha amylase composition or mixture, produce a high dextrose product. In some embodiments, the yield of glucose produced by the liquefaction and saccharification (glucose percent of the total hydrolyzed and solublized dry solids) is at least 70%, at least 80%, at least 85%, at least 90%, at least 92% and at least 95%.

In some embodiments, the glucose produced may be further used to produce fructose or high purity dextrose. In some embodiments of the invention, glucose will be separated from the treated mash by methods known in the art such as by centrifugation, membrane separation and conventional filtration methods. The glucose may be enzymatically converted to fructose syrup by means known in the art.

In some embodiments, the method further comprises using the dextrin (e.g. glucose) as a fermentation feedstock in microbial fermentations under suitable fermentation conditions to obtain end-products, such as alcohol (e.g., ethanol), organic acids (e.g., succinic acid, lactic acid), sugar alcohols (e.g., glycerol), ascorbic acid intermediates (e.g., gluconate, DKG, KLG) amino acids (e.g., lysine), and proteins (e.g., antibodies and fragments thereof).

The organism used in fermentations will depend on the desired end product. Typically if ethanol is the desired end product, yeast will be used as the fermenting organism. In some embodiments, the ethanol-producing microorganism is a yeast and specifically *Saccharomyces* such as strains of *S. cerevisiae* (USP 4,316,956). A variety of *S. cerevisiae* are commercially available and these include but are not limited to FALI (Fleischmann's Yeast), SUPERSTART (Alltech), FERMIOL (DSM Specialties), RED STAR (Lesaffre) and Angel alcohol yeast (Angel Yeast Company, China). The amount of starter yeast employed in the methods is an amount effective to produce a commercially significant amount of ethanol in a suitable amount of time, (*e.g.* to produce at least 10% ethanol from a substrate having between 25 to 40% DS in less than 72 hours). Yeast cells are generally supplied in amounts of 10⁴ to 10¹², and preferably from 10⁷ to 10¹⁰ viable yeast count per ml of fermentation broth. The fermentation can include, in addition to fermenting microorganisms (e.g. yeast), nutrients, acid and/or additional enzymes, including but not limited to phytases.

The use of yeast in fermentations is well known and reference is made to THE ALCOHOL TEXTBOOK, K. JACQUES ET AL., EDS. 1999, NOITINGHAM UNIVERSITY PRESS, UK. In some embodiments, the amount of ethanol produced by the methods encompassed by the invention will be at least 8%, at least 10%, at least 12%, at least 14%, at least 15%, at least 16%, at least 17%, at least 18%, at least 20% and at least 22% (v/v).

Optionally, following fermentation, alcohol (e.g. ethanol) can be extracted by, for example, distillation. Ethanol can be used for fuel, portable or industrial ethanol.

In some embodiments, the use of phytase compositions during starch hydrolysis may reduce the phytic acid content of the fermentation broth, the phytate content of the thin stillage and/or the phytic acid content of co-products of the fermentation such as Distillers Dried Grains (DDG); Distillers Dried Grains with Solubles (DDGS); Distillers wet grains (DWG) and Distillers wet grains with solubles (DWGS). In some embodiments, the methods of the invention (including but not limited to for example incubation of 30 to 60 minutes) can reduce the phytic acid content of the resulting fermentation filtrate by at least 60%, 65%, 70%, 75%, 80%, 85% and 90% and greater as compared to essentially the same process without the phytase. In some embodiments, the amount of phytate found in the DDGS may be reduced by at least 50%, at least 70%, at least 80% and at least 90% as compared to the phytate content in DDGS from a corresponding process which is essentially the same as the claimed process, but without a phytase according to the invention. For example, while the % phytate content in commercial samples of DDGS may vary, a general range of % phytate may be about 1% to about 3% or higher. In some embodiments, the % phytate in the DDGS obtained from the current process will be less than 1.0%, less than 0.8%, less than 0.5% and also less than 0.3%. In some embodiments, the amount of phytate found in the thin stillage may be reduced by at least 50%, at least 70%, at least 80% and at least 90% as compared to the phytate content in thin stillage from a corresponding process which is essentially the same as the claimed process but without a phytase according to the invention.

In industrial ethanol processes, ethanol may be distilled from the filtrate resulting in a thin stillage portion that may be and frequently is recycled into the fermentation stream (backset). The present invention results in thin stillage having a lower phytate content as compared to the phytate content of thin stillage from a corresponding process which is essentially the same as the claimed process but without a phytase encompassed by the invention. For example, the amount of phytate (ppm) in thin stillage having about 8% ds may be in the range of 2500 to 3000 ppm. In some embodiments of the present invention, the phytate content in thin stillage may be less than 2000 ppm, less than 1000 ppm, less than 500 ppm, less than 100 ppm and less than 50 ppm when the substrate has been treated according to the methods encompassed herein.

### EXPERIMENTAL

The present invention is described in further detail in the following examples which are not in any way intended to limit the scope of the invention as claimed. The attached Figures are meant to be considered as integral parts of the specification and description of the invention. The following examples are offered to illustrate, but not to limit the claimed invention.

In the disclosure and experimental section which follows, the following abbreviations apply: wt% (weight percent); °C (degrees Centigrade); H₂O (water); dH₂O (deionized water); dIH₂O (deionized water, Milli-Q filtration); g or gm (grams); µg (micrograms); mg (milligrams); kg (kilograms); µL (microliters); ml and mL (milliliters); mm (millimeters); µm (micrometer); M (molar); mM (millimolar); M (micromolar); U (units); MW (molecular weight); sec (seconds); min(s) (minute/minutes); hr(s) (hour/hours); ds (dry solids); DO (dissolved oxygen); WN (weight to volume); W/W (weight to weight); VN (volume to volume); Genencor (Genencor International, Inc., Palo Alto, CA); IKA (IKA Works Inc. 2635 North Chase Parkway SE, Wilmington, NC); Genencor (Danisco US Inc, Genencor Division, Palo Alto, CA); MT (Metric ton); Ncm (Newton centimeter); ETOH (ethanol); eq (equivalents); N (Normal); ds or DS (dry solids content); /g ds (per gram dry solids); SAPU (spectrophotometer protease unit).

### Methods

Viscosity Measurements: A glass cooker-viscometer, LR-2.ST system IKA was used to determine viscosity. In brief the viscometer consists of a 2000 ml double walled glass vessel with an anchor mixer that is stirred by a Eurostar Labortechnik power control-viscometer (the viscosity range of the Viscoklick viscometer is 0-600 Ncm). In general for the examples described herein a slurry comprising starch substrate and an appropriate amount of enzyme was poured into the viscometer vessel. The temperature and viscosity were recorded during heating to 85°C and incubation was continued for additional 60 to 120 mins. Viscosity measured as Ncm was recorded at intervals.

Carbohydrate and Alcohol Analysis by High Pressure Liquid Chromatographic (HPLC): The composition of the reaction products of oligosaccharides was measured by HPLC (Beckman System Gold 32 Karat Fullerton, CA equipped with a HPLC column (Rezex 8 u8% H, Monosaccharides), maintained at 50°C fitted with a refractive index (RI) detector (ERC-7515A RI Detector, Anspec Company Inc.). Saccharides were separated based on molecular weight. A designation of DP1 is a monosaccharide, such as glucose; a designation of DP2 is a disaccharide, such as maltose; a designation of DP3 is a trisaccharide, such as maltotriose and the designation "DP4⁺" is an oligosaccharide having a degree of polymerization (DP) of 4 or greater.

Phytase Activity (FTU) was measured by the release of inorganic phosphate. The inorganic phosphate forms a yellow complex with acidic molybdate/vandate reagent and the yellow complex was measured at a wavelength of 415 nm in a spectrophometer and the released inorganic phosphate was quantified with a phosphate standard curve. One unit of phytase (FTU) is the amount of enzyme that releases 1 micromole of inorganic phosphate from phytate per minute under the reaction conditions given in the European Standard (CEN/TC 327,2005-TC327WI 003270XX).

Phytic acid content: - Phytic acid was extracted from a sample by adjusting the pH of a 5% slurry (for dry samples) to pH 10.0 and then determined by HPLC using an ion exchange column. Phytic acid was eluted from the column using a NaOH gradient system. Phytic acid content in the liquid was calculated by comparing phytic acid to a standard.

Alpha amylase activity (AAU) can be determined by the rate of starch hydrolysis, as reflected in the rate of decrease of iodine-staining capacity measured spectrophotometrically. One AAU of bacterial alpha-amylase activity is the amount of enzyme required to hydrolyze 10 mg of starch per min under standardized conditions.

Alpha-amylase activity can also be determined as soluble starch unit (SSU) and is based on the degree of hydrolysis of soluble potato starch substrate (4% DS) by an aliquot of the enzyme sample at pH 4.5, 50°C. The reducing sugar content is measured using the DNS method as described in Miller, G. L. (1959) Anal. Chem. 31:426 - 428.

Alpha amylase activity in Liquifon Units (LU) for SPEZYME FRED was measured according to the method disclosed in USP 5,958,739. In brief, the assay method uses p-nitrophenyl maltoheptoside as a substrate with the non-reducing terminal sugar chemically blocked. The rate of p-nitrophenyl release is proportional to alpha amylase activity and release is monitored at 410 nm. Activity is calculated against a standard control.

Glucoamylase Activity Units (GAU) is determined by using the PNPG assay to measure the activity of glucoamylase.

### EXAMPLES

### Example 1: SPEZYME XTRA and BP-WT

An aqueous slurry (1.0 kgs) of whole ground corn (36% ds, 800 g ground corn) was prepared. The pH of the slurry was adjusted to pH 5.8 using dilute H₂SO₄. The slurry was transferred to a flask and SPEZYME XTRA (Genencor) was added at 2.8 AAU/gds (+ or - BP-WT (SEQ ID NO:1) at 7.2 FTU g ds or SPEZYME XTRA was added without phytase at 5.6AAU/gds and in all cases the slurry was incubated at 65° C for 30 min.

The preheated whole ground corn slurry was transferred to a holding vessel in the viscometer (IKA). The slurry was continuously heated until reaching 85°C and then the flurry was maintained at this temperature. The viscosity of the slurry was measured as temperature was increased (Table 4).

**Table 4**

| | SPEZYME XTRA | | SPEZYME XTRA | | SPEZYME XTRA | |
|---|---|---|---|---|---|---|
| | 2.8 AAU/gds | | 2.8 AAU/gds + Phytase 7.2 FTU gds | | 5.6 AAU/gds | |
| | | Viscosity | | Viscosity | | Viscosity |
| Time (min) | °C | Ncms | °C | Ncms | °C | Ncms |
| 0.0 | 65.2 | 8.6 | 65.0 | 7.4 | 65.0 | 7.7 |
| 0.5 | 66.6 | 9.3 | 66.5 | 9.6 | 66.1 | 8.1 |
| 1.0 | 68.0 | 12.7 | 68.0 | 16.0 | 67.4 | 10.3 |
| 1.5 | 69.0 | 21.7 | 69.0 | 28.7 | 68.7 | 15.7 |
| 2.0 | 70.0 | 41.0 | 69.8 | 39.2 | 69.7 | 30.6 |
| 2.5 | 70.4 | 50.1 | 70.3 | 43.3 | 70.4 | 38.3 |
| 3.0 | 70.9 | 54.5 | 71.2 | 49.5 | 71.1 | 40.9 |
| 3.5 | 71.6 | 58.3 | 71.9 | 52 | 71.9 | 43.6 |
| 4.0 | 72.3 | 63.4 | 72.5 | 55.0 | 72.7 | 44.4 |
| 4.5 | 72.8 | 67.8 | 73.2 | 57.7 | 73.3 | 43.3 |
| 5.0 | 72.9 | 68.1 | 73.7 | 58.0 | 74.2 | 43.4 |
| 5.5 | 73.5 | 66.2 | 74.7 | 53.9 | 75.1 | 42.1 |
| 6.0 | 74.3 | 64.0 | 76.1 | 51.4 | 76.0 | 39.8 |
| 6.5 | 75.2 | 61.3 | 77.0 | 48.2 | 76.8 | 37.3 |
| 7.0 | 76.4 | 58.5 | 77.6 | 44.6 | 77.7 | 34.8 |
| 7.5 | 77.8 | 55.2 | 78.3 | 41.0 | 78.4 | 33.0 |
| 8.0 | 79.2 | 53.0 | 79.3 | 36.8 | 79.1 | 31.0 |
| 8.5 | 80.0 | 49.9 | 79.8 | 34 | 80.1 | 29.6 |
| 9.0 | 80.4 | 47.9 | 80.6 | 30.4 | 80.7 | 28.3 |
| 9.5 | 81.1 | 45.2 | 81.2 | 29.2 | 81.4 | 27.1 |
| 10.0 | 81.5 | 44.1 | 81.8 | 27.8 | 81.9 | 26.3 |
| 14.0 | 84.1 | 38.5 | 83.9 | 19.9 | 84.6 | 25.8 |
| 18.0 | 84.3 | 37.6 | 84.1 | 15.9 | 84.8 | 24.3 |
| 22.0 | 84.0 | 37.5 | 84.0 | 13.6 | 84.2 | 24.3 |
| 26.0 | 84.4 | 37.4 | 84.3 | 12.5 | 84.4 | 24.0 |
| 30.0 | 84.9 | 36.5 | 84.8 | 11.6 | 84.8 | 23.8 |
| 34.0 | 85.3 | 36.3 | 85.3 | 10.6 | 85.3 | 23.7 |
| 38.0 | 85.5 | 35.8 | 85.4 | 9.8 | 85.3 | 23.8 |
| 42.0 | 85.6 | 35.8 | 85.7 | 9.0 | 85.3 | 23.8 |
| 48.0 | 85.7 | 35.6 | 84.8 | 8.8 | 85.4 | 23.7 |
| 50.0 | 85.8 | 35.5 | 84.6 | 8.6 | 85.4 | 23.9 |
| 55.0 | 85.8 | 35.2 | 85.6 | 8.4 | 85.4 | 23.8 |
| 58.0 | 85.6 | 35.4 | 86.0 | 8.2 | 85.4 | 23.8 |
| 62.0 | 85.3 | 36.2 | 86.1 | 7.7 | 85.4 | 23.7 |

The viscosity data in Table 4 illustrates that the incubation of whole ground corn at 85°C with a standard dose of SPEZYME XTRA (5.6 AAU/gds) produced the liquefact with a viscosity stabilized at 23.8 Ncm. In contrast, the pre-treatment of whole ground corn with half the dose of SPEZYME XTRA (2.8 AAU/g ds) and 7.2 FTU of BP-WT resulted in a lower viscosity of the liquefact at 85°C.

### Example 2: SPEZYME ETHYL and BP-WT

The effect of SPEZYME ETHYL was studied during the incubation of whole ground corn with *Buttiauxella* phytase (BP-WT). The conditions were the same as described in example 1, at pH 5.8. In addition, the incubation of whole ground corn was carried out at a lower pH, (pH 4.5, 65°C) prior to raising the temperature to 85°C. Starting at time 0, samples were taken every 30 sec for the first 10 mins and every 4 minutes thereafter until 62 mins. Some of the results are summarized in Table 5A, (pH 5.8, 65°C 30 min incubation) and Table 5B (pH 4.5, 65°C 30 min incubation).

**Table 5A - Effect of phytase on viscosity reduction at 85°C during incubation of whole ground corn (35% ds) with SPEZYME ETHYL**

| **SPEZYME ETHYL (5.6 AAU/gds)** | | **SPEZYME ETHYL (2.8 AAU/gds +Phytase (7.2 FTU/g ds)** | |
|---|---|---|---|
| °C | Ncms | °C | Ncms |
| 65.0 | 9.6 | 65.0 | 9.3 |
| 66.3 | 11.2 | 66.0 | 9.4 |
| 67.8 | 16.0 | 67.2 | 11.0 |
| 68.9 | 24.4 | 68.2 | 15.0 |
| 69.8 | 41.2 | 69.6 | 31.8 |
| 70.3 | 46.6 | 70.4 | 50.6 |
| 71.1 | 49.7 | 70.7 | 58.3 |
| 71.7 | 52.9 | 71.4 | 54.0 |
| 72.3 | 55.3 | 72.0 | 69.5 |
| 73.0 | 57.0 | 72.4 | 78.2 |
| 73.6 | 55.2 | 72.9 | 8.10 |
| 74.8 | 52.8 | 73.7 | 81.3 |
| 76.2 | 49.9 | 74.8 | 78.1 |
| 77.0 | 46.7 | 75.7 | 73.2 |
| 77.7 | 43.7 | 76.4 | 67.7 |
| 78.4 | 39.1 | 77.2 | 61.8 |
| 79.1 | 36.0 | 77.9 | 55.8 |
| 79.9 | 32.6 | 79.4 | 51.8 |
| 80.5 | 30.5 | 80.7 | 47.9 |
| 81.5 | 27.7 | 81.3 | 44.1 |
| 81.9 | 26.3 | 81.8 | 41.2 |
| 84.2 | 20.0 | 85.3 | 26.5 |
| 84.2 | 18.1 | 85.0 | 21.9 |
| 83.9 | 16.9 | 84.4 | 18.9 |
| 84.2 | 16.1 | 84.5 | 16.1 |
| 84.8 | 15.2 | 84.7 | 14.4 |
| 84.8 | 14.8 | 84.9 | 13.0 |
| 84.4 | 13.7 | 85.1 | 11.8 |
| 84.3 | 13.3 | 85.3 | 10.8 |
| 84.5 | 13.1 | 85.5 | 10.2 |
| 85.0 | 13.2 | 85.5 | 9.7 |
| 85.2 | 13.0 | 85.5 | 9.2 |
| 85.3 | 12.9 | 85.4 | 8.8 |
| 85.3 | 12.7 | 85.2 | 8.6 |
| 85.5 | 12.5 | 85.5 | 7.5 |
| 86.0 | 12.4 | 85.4 | 7.1 |

**Table 5B - Effect of phytase on viscosity reduction at 85°C during incubation of whole ground corn (35% ds) with SPEZYME ETHYL**

| **SPEZYME ETHYL (1.85 AAU/g ds)** | | **SPEZYME ETHYL (1.85 AAU/g ds)** | | **SPEZYME ETHYL (5.6 AAU/gds)** | | **SPEZYME ETHYL (5.6 AAU/gds)** | |
|---|---|---|---|---|---|---|---|
| | | **+Phytase (7.2 FTU/gds** | | | | **+Phytase (7.2 FTU/gds)** | |
| °C | Ncms | °C | Ncms | °C | Ncms | °C | Ncms |
| 59.9 | 5.7 | 65.0 | 9.0 | 65.0 | 10.9 | 65.0 | 8.9 |
| 61.4 | 5.8 | 66.2 | 9.7 | 66.1 | 11.6 | 66.0 | 9.2 |
| 62.8 | 5.8 | 67.5 | 11.6 | 67.3 | 13.8 | 67:2 | 10.8 |
| 64.5 | 6.4 | 68.6 | 17.1 | 68.5 | 18.8 | 68.5 | 14.3 |
| 65.8 | 8.5 | 70.2 | 47.4 | 69.8 | 36.7 | 69.7 | 22.9 |
| 66.3 | 10.6 | 70.6 | 59.9 | 70.6 | 52.9 | 70.7 | 38.9 |
| 67.4 | 18.9 | 71.1 | 68.9 | 71.0 | 60.2 | 71.4 | 46.3 |
| 68.6 | 42.1 | 71.6 | 77.0 | 71.8 | 66.9 | 72.0 | 49.3 |
| 69.3 | 65.8 | 72.5 | 90.3 | 72.4 | 72.3 | 72.6 | 50.8 |
| 69.8 | 91.6 | 73.1 | 99.6 | 73.0 | 82.8 | 73.3 | 54.6 |
| 70.5 | 124.8 | 73.5 | 114.9 | 73.5 | 89.8 | 73.8 | 56.2 |
| 71.0 | 170.0 | 73.9 | 128.5 | 74.0 | 95.9 | 74.6 | 57.0 |
| 71.5 | 215.0 | 74.6 | 132.9 | 74.5 | 98.7 | 75.4 | 55.9 |
| 71.8 | 291.0 | 75.4 | 130.1 | 75.3 | 98.9 | 76.5 | 54.8 |
| 71.6 | 360.0 | 75.9 | 126.2 | 76 | 96.5 | 77.7 | 52.6 |
| 72.0 | 405.0 | 76.4 | 119.9 | 76.9 | 94.2 | 78.7 | 50.3 |
| | | 77.2 | 114.1 | 77.3 | 92.6 | 79.6 | 47.7 |
| | | 77.9 | 108.3 | 78.2 | 90.2 | 80.3 | 45.1 |
| | | 78.9 | 102.8 | 79.0 | 89.0 | 80.9 | 43.4 |
| | | 79.8 | 98.0 | 79.8 | 88.1 | 81.5 | 41.5 |
| | | 80.4 | 94.9 | 80.5 | 86.4 | 82.1 | 39.9 |
| | | 84.3 | 74.0 | 84.9 | 87.3 | 84.7 | 33.3 |
| | | 85.3 | 67.3 | 86.0 | 86.6 | 84.7 | 31.9 |
| | | 85.1 | 64.2 | 85.6 | 88.2 | 84.3 | 31.6 |
| | | 84.9 | 61.7 | 84.9 | 89.9 | 84.5 | 31.7 |
| | | 84.9 | 60.3 | 84.4 | 90.4 | 84.8 | 31.6 |
| | | 85.0 | 59.5 | 84.2 | 90.2 | 85.1 | 31.0 |
| | | 85.1 | 59.2 | 84.6 | 90.1 | 84.8 | 31.5 |
| | | 84.7 | 59.0 | 85.0 | 89.6 | 84.5 | 31.6 |
| | | 84.6 | 58.9 | 85.5 | 89.6 | 84.7 | 31.7 |
| | | 84.8 | 58.9 | 85.3 | 90.2 | 85 | 31.5 |
| | | 84.9 | 58.3 | 84.7 | 91.3 | 85.2 | 31.6 |

### Example 3: SPEZYME FRED and BP-WT

The effect of SPEZYME FRED was studied during the incubation of whole ground corn with *Buttiauxella* phytase (BP-WT). The conditions were the same as described in example 1 (pH 5.8, 65°C 30 min incubation) prior to raising the temperature to 85°C. Starting at time 0, samples were taken every 30 sec for the first 10 mins and every 4 minutes thereafter until 62 mins. Results are summarized in Table 6.

**Table 6 - Effect of phytase on viscosity reduction at 85°C during incubation of whole ground corn (35% ds) with SPEZYME FRED**

| SPEZYME FRED, pH 5.8 10 LU/gds, 65°C, 30 min ( - phytase) | | SPEZYME FRED, pH 5.8 10 LU/gds, 65°C, 30 min ( + BP-WT phytase 7.2 FTU/g ds)) | | SPEZYME FRED, pH 5.8 20 LU/gds, 65°C, 30 min (- phytase) | |
|---|---|---|---|---|---|
| °C | Ncms | °C | Ncms | °C | Ncms |
| 65.3 | 8.4 | 65.0 | 7.5 | 65.2 | 7.9 |
| 66.4 | 8.7 | 66.3 | 8.7 | 67.1 | 10.9 |
| 67.7 | 12.4 | 67.6 | 11.6 | 68.0 | 14.7 |
| 68.8 | 36.7 | 68.9 | 29.3 | 69.0 | 38.7 |
| 69.5 | 61.5 | 69.7 | 62.8 | 69.6 | 56.0 |
| 70.0 | 82.3 | 70.0 | 76.8 | 70.1 | 62.9 |
| 70.6 | 98.9 | 70.8 | 92.9 | 70.7 | 70.2 |
| 71.1 | 118.6 | 71.6 | 104.5 | 71.3 | 77.0 |
| 71.7 | 135.5 | 72.0 | 116.4 | 71.8 | 84.9 |
| 72.2 | 146.9 | 72.6 | 122.6 | 72.4 | 87.6 |
| 73.1 | 138.8 | 73.0 | 122.0 | 73.0 | 86.6 |
| 74.0 | 128.9 | 73.6 | 114.6 | 73.7 | 82.4 |
| 74.7 | 117.5 | 74.1 | 103.5 | 74.7 | 77.1 |
| 75.5 | 105.3 | 74.7 | 95.6 | 75.5 | 70.3 |
| 75.9 | 93.8 | 75.8 | 87.3 | 76.3 | 63.4 |
| 76.4 | 84.1 | 76.8 | 78.2 | 76.8 | 56.6 |
| 77.5 | 77.3 | 77.8 | 70.8 | 78.7 | 51.6 |
| 78.6 | 70.7 | 78.6 | 65.3 | 79.9 | 47.5 |
| 79.3 | 65.1 | 79.3 | 60.4 | 80.3 | 42.7 |
| 80.0 | 60.7 | 80.9 | 54.5 | 80.8 | 39.5 |
| 80.5 | 56.5 | 82.1 | 51.0 | 81.6 | 35.3 |
| 85.0 | 35.8 | 84.7 | 31:3 | 84.4 | 23.5 |
| 85.6 | 29.2 | 85.3 | 23.3 | 84.6 | 18.5 |
| 84.6 | 26.1 | 84.7 | 19.2 | 84.3 | 15.0 |
| 83.8 | 23.9 | 84.4 | 17.1 | 84.6 | 13.4 |
| 84.0 | 22.0 | 84.6 | 15.0 | 85.2 | 12.0 |
| 84.2 | 21.2 | 85.0 | 13.1 | 85.6 | 11.3 |
| 84.3 | 20.4 | 85.4 | 11.6 | 85.5 | 10.3 |
| 84.2 | 19.5 | 85.4 | 10.5 | 85.4 | 9.9 |
| 84.5 | 18.8 | 85.4 | 10.3 | 85.5 | 9.5 |
| 84.6 | 17.8 | 85.5 | 9.2 | 85.6 | 9.2 |
| 84.5 | 17.3 | 85.4 | 8.8 | 85.5 | 9.0 |
| 84.4 | 117.2 | 85.4 | 8.7 | 85.4 | 9.1 |
| 85.9 | 16.3 | 85.4 | 8.1 | 85:1 | 8.7 |

The results illustrated in Table 6 demonstrate that incubation at pH 5.8, 65°C for 30 min with phytase and SPEZYME FRED resulted in a liquefact having reduced viscosity.

### Example 4: Viscosity effects in the presence of BP-WT

The pH of a slurry comprising 36% whole ground corn was adjusted to pH 5.8, pH 5.4, pH 5.2 or pH 5.0 using dilute HCL. SPEZYME XTRA (2.0 AAU/g ds) and BP-WT (3.6 FTU/g ds) were added to the slurry and maintained at 65°C for 30 mins. SPEZYME XTRA (4.0AAU/g ds at pH 5.8) without phytase was used as a control. The viscosity of the slurry was measured during heating to 85°C (Table 7).

**Table 7**

| | SPEZYME XTRA (4 AAU/gds) | SPEZYME XTRA (2 AAU/gds) + BP-WT | SPEZYME XTRA (2 AAU/gds) + BP-WT | SPEZYME XTRA (2 AAU/gds) + BP-WT |
|---|---|---|---|---|
| | pH 5.8 | pH 5.8 | pH 5.4 | pH 5.2 |
| | (Ncms) | (Ncms) | (Ncms) | (Ncms) |
| Time (min) | | | | |
| 0 | 7.4 | 8.4 | 6.3 | 7.4 |
| 0.5 | 9.2 | 8.5 | 6.5 | 9.2 |
| 1.0 | 12.9 | 10.0 | 8.8 | 12.9 |
| 1.5 | 26.6 | 15.4 | 15.0 | 26.6 |
| 2.0 | 40.0 | 32.5 | 22.2 | 40.0 |
| 2.5 | 48.2 | 42.4 | 34.3 | 48.2 |
| 3.0 | 50.9 | 47.9 | 43.7 | 50.9 |
| 3.5 | 54.6 | 51.1 | 48.7 | 54.6 |
| 4.0 | 58.3 | 54.1 | 52.5 | 58.3 |
| 4.5 | 60.9 | 57.1 | 55.6 | 60.9 |
| 5.0 | 61.2 | 58.4 | 58.5 | 61.2 |
| 5.5 | 58.2 | 56.5 | 59.2 | 58.2 |
| 6.0 | 54.7 | 53.3 | 56.2 | 54.7 |
| 6.5 | 51.4 | 50.1 | 53.8 | 51.4 |
| 7.0 | 48.4 | 46.7 | 49.9 | 48.4 |
| 7.5 | 45.4 | 43.3 | 46.2 | 45.4 |
| 8.0 | 41.9 | 39.9 | 43.1 | 41.9 |
| 8.5 | 38.5 | 36.5 | 39.9 | 38.5 |
| 9.0 | 36.8 | 33.9 | 36.0 | 36.8 |
| 9.5 | 34.3 | 31.5 | 34.3 | 34.3 |
| 10 | 32.6 | 29.7 | 31.8 | 32.6 |
| 14 | 24.8 | 21.6 | 23.6 | 24.8 |
| 18 | 22.6 | 17.9 | 20.5 | 22.6 |
| 22 | 21.2 | 16.7 | 18.4 | 21.2 |
| 26 | 20.8 | 14.0 | 17.6 | 20.8 |
| 30 | 20.0 | 12.7 | 16.5 | 20.0 |
| 34 | 19.5 | 12.1 | 15.9 | 19.5 |
| 38 | 19.2 | 11.6 | 15.7 | 19.2 |
| 42 | 19.1 | 11.0 | 15.4 | 19.1 |
| 46 | 19.2 | 10.9 | 15.3 | 19.2 |
| 50 | 19.1 | 10.9 | 15.3 | 19.1 |
| 54 | 18.8 | 10.7 | 15.2 | 18.8 |
| 58 | 18.6 | 10.6 | 15.0 | 18.6 |
| 62 | 17.7 | 10.5 | 14.9 | 17.7 |

Table 7 illustrates that at ph 5.8, 5.4 and 5.2, the reduction in viscosity of the slurry using BP-WT and SPEZYME XTRA (2.0 AAU/gds) was comparable to the reduction in viscosity of the slurry with the control SPEZYME XTRA (4..0 AAU/gds) at pH 5.8. However, the dose of alpha amylase in the combination was half of the dose in the control. Data for pH 5.0 is not shown. The combination of SPEZYME XTRA and BP-WT did not reduce the viscosity of the slurry under the conditions of the test relative to the control.

### Example 5: Effect on thin stillage and DDGS

A whole ground corn slurry (36% ds) was incubated at 65°C for 30 min, 60 min or 120 min in the presence of 2.0 AAU of SPEZYME XTRA and 3.6 FTU of *Buttiauxella* Phytase -WT. Conditions were as described above in example 1. After the specified time, the temperature was increased to 85°C and held at this temperature for 60 min. The pH of the liquefied starch substrate was reduced to pH 4.2 and further evaluated under yeast fermentation conditions. The %ds of the liquefied starch was adjusted to 3!% ds. FERMENZYME™ L-400 was added at 0.4 GAU/gds and using yeast fermentation was carried out at 32°C. The final alcohol concentration, residual starch content and phytic acid content of the thin stillage and DDGS were determined (Table 8).

**Table 8**

| Incubation at 65C, pH 5.8 with 2.0AAU SPEZYME XTRA | Heating at 85°C | Yeast Fermentation | | | |
|---|---|---|---|---|---|
| (min) | (min) | % alcohol (V/V) (60 hrs) | % Residual Starch in DDGS | Phytic acid (ppm) 60 hrs, Culture filtrate | % Phytic acid in DDGS |
| Control ( minus BP-WT | 60 | 14.73 | 9.77 | 480 | 1.02 |
| 30 | 60 | 14.72 | 7.55 | 56 | 0.21 |
| 60 | 60 | 14.72 | 8.36 | 60 | 0.18 |
| 120 | 60 | 14.37 | 7.55 | 40 | 0.21 |

As observed from Table 8, an incubation time of only 30 minutes can significantly reduce the phytic acid content of the resulting fermentation filtrate. The filtrate of a standard control (without phytase) was measured at 480 ppm compared with only 56 ppm in the filtrate of the incubated samples for 30 mins and 40 ppm in the filtrate of the incubated samples for 120 mins.

### Example 6: Production of glucose from liquefied starch - BP-WT

A 36% ds whole ground corn slurry was incubated at 65°C for 30 min in the presence of SPEZYME XTRA (2.0 AAU) and 3.6 FTU of *Buttiauxella* Phytase-WT under the same conditions as described in example 1. After 30 mins, the temperature was increased to 85°C and held at 85°C for an additional 60 min. The temperature of the liquefact was reduced to 60°C and the pH was adjusted to pH 4.2. The liquefied starch was adjusted to 32% and 36% ds using H₂O and saccharified at 60°C using OPTIMAX™ 4060 VHP at a dose of 0.4 Kgs/MT of ds corn. Samples were taken at different intervals of time during incubation at 60 °C and analyzed for glucose yield using HPLC (Table 9).

**Table 9 - Production of glucose from liquefied starch**

| 32% ds | | | | |
|---|---|---|---|---|
| Hrs | % glucose | % DP2 | % DP3 | % DP4 |
| 16 | 90.5 | 3.7 | 1.9 | 3.9 |
| 24 | 93.6 | 3.0 | 1.8 | 1.6 |
| 40 | 94.7 | 2.9 | 1.7 | 0.7 |

| 36% ds | | | | |
|---|---|---|---|---|
| Hrs | | | | |
| 16 | 89.3 | 3.9 | 2.0 | 4.8 |
| 24 | 94.4 | 2.8 | 1.4 | 1.4 |
| 40 | 94.5 | 3.0 | 1.7 | 0.7 |

The resulting high dextrose product may be used as a feedstock in the fermentation process for the production of end products including but not limited to alcohol, organic acids, amino acids, ascorbic acid intermediates, sugar alcohols and the like.

### Example 7: Effect of phytase incubation on viscosity

The effect of phytase incubation on viscosity reduction at an elevated temperature of 85°C was studied under various conditions when a slurry comprising whole ground corn (36% ds), at pH 5.8, 65°C was incubated with BP-WT (3.6 FTU/g ds).

Condition 1, is a control wherein phytase was not added and SPEZYME XTRA (4 AAU/gds) was added to the incubation which was only 30 minutes; Condition 2, BP-WT added at the beginning of the incubation and the slurry was incubated for 60 mins. At about 60 mins SPEZYME XTRA (2 AAU/gds) added to the slurry; Condition 3, both BP-WT and SPEZYME XTR A (2 AAU/gds) were added at the beginning of the 60 min incubation; and Condition 4, SPEZYME XTRA (2 AAU/gds) was added at the beginning of the incubation and the incubation was for 30 mins. In all cases, the temperature was elevated to 85°C after the incubation period. Viscosity was measured over time.

**Table 10 - Comparison of phytase incubation on DE and solubilization of starch (BRIX)**

| | **Incubation BP-WT + SPEZME XTRA** | | **Incubation BP-WT only** | |
|---|---|---|---|---|
| **Time (min)** | **DE** | **BRIX** | **DE** | **BRIX** |
| 30 | 9.97 | 30.7 | 9.20 | |
| 60 | 10.11 | 31.3 | 9.12 | |
| 90 | 10.57 | 31.4 | 8.73 | |
| 120 | 10.60 | 31.6 | 8.93 | 31.4 |

**Table 11**

| Time (mins) | Condition 1 (°C) (Ncms) | | Condition 2 (°C) (Ncms) | | Condition 3 (°C) (Ncms) | | Condition 4 (°C) (Ncms) | |
|---|---|---|---|---|---|---|---|---|
| 0 | 65.0 | 6.0 | 65.6 | 7.2 | 67.0 | 5.2 | 65.2 | 8.6 |
| 0.5 | 65.5 | 6.8 | 66.7 | 6.9 | 68.1 | 5.1 | 66.6 | 9.3 |
| 1.0 | 65.9 | 10.2 | 67.6 | 8.1 | 69.2 | 5.6 | 68.0 | 12.7 |
| 2.0 | 68.8 | 24.9 | 69.3 | 28.0 | 71.1 | 30.4 | 70.0 | 41.0 |
| 3.0 | 70.5 | 40.0 | 70.2 | 56.2 | 72.1 | 58.5 | 70.9 | 54.5 |
| 4.0 | 72.0 | 45.9 | 71.7 | 65.2 | 73.2 | 64.9 | 72.3 | 63.4 |
| 5.0 | 73.6 | 47.6 | 72.7 | 73.7 | 74.2 | 61.1 | 72.9 | 68.1 |
| 6.0 | 75.3 | 45.1 | 74.3 | 67.5 | 76.5 | 54.3 | 74.3 | 64.0 |
| 7.0 | 76.9 | 39.9 | 76.1 | 57.0 | 78.9 | 46.0 | 76.4 | 58.5 |
| 8.0 | 78.4 | 34.2 | 77.9 | 49.8 | 80.0 | 38.4 | 79.2 | 53.0 |
| 9.0 | 80.2 | 29.8 | 80.2 | 40.8 | 81.1 | 33.2 | 80.4 | 47.9 |
| 10.0 | 81.5 | 27.5 | 81.1 | 36.1 | 82.4 | 29.6 | 81.5 | 44.1 |
| 14.0 | 84.3 | 23.9 | 84.7 | 25.4 | 84.2 | 21.8 | 84.1 | 38.5 |
| 22.0 | 84.1 | 23.8 | 84.6 | 19.7 | 84.0 | 17.7 | 84.0 | 37.5 |
| 26.0 | 84.3 | 23.6 | 84.5 | 18.4 | 84.6 | 16.5 | 84.4 | 37.4 |
| 30.0 | 84.6 | 23.9 | 84.6 | 17.5 | 85.3 | 15.7 | 84.9 | 36.5 |
| 34.0 | 85.0 | 23.9 | 84.8 | 17.0 | 85.2 | 15.2 | 85.3 | 36.3 |
| 42.0 | 85.0 | 23.5 | 85.0 | 16.7 | 84.9 | 14.8 | 85.6 | 35.8 |
| 46.0 | 85.1 | 23.5 | 85.5 | 16.0 | 85.1 | 14.4 | 85.7 | 35.6 |
| 50.0 | 85.3 | 23.3 | 85.7 | 15.9 | 85.4 | 14.2 | 85.8 | 35.5 |
| 54.0 | 85.4 | 23.4 | 85.6 | 15.9 | 85.4 | 14.2 | 85.8 | 35.2 |
| 62.0 | 85.2 | 23.5 | 85.4 | 15.6 | 85.3 | 14.1 | 85.3 | 36.2 |

As shown in Tables 10 and 11, at a gelatinization temperature of about 72 - 74°C, the peak viscosity was lowered by the higher dose of SPEZYME XTRA (4 AAU/gds as opposed to 2AAU/gds). However, the incubation with BP-WT with or without SPEZYME XTRA resulted in a significant reduction in the viscosity at 85°C even with a 50% reduced dose of SPEZYME XTRA during the elevated heating step at 85°C

### Example 8: Mixtures of alpha amylases

A 36% ds slurry of whole ground corn was incubated at pH 5.8 for 30 mins, 65°C. The slurry was contacted with one of the treatments indicated below, heated and maintained at 85°C. Viscosity measurements were made every 10 - 30 seconds during the pretreatment, temperature increase and temperature hold steps.
Treatment 1- SPEZYME FRED (10 LU) + SPEZYME XTRA (2AAU);
Treatment 2 - SPEZYME FRED (5 LU) + SPEZYME XTRA (1AAU) + BP-WT (7.6 FTU/gds;
Treatment 3 - SPEZYME FRED (2.5 LU) + SPEZYME XTRA (2 AAU) + BP-WT (7.6 FTU/gds);
Treatment 4 - SPEZYME XTRA (2 AAU) + BP-Wt (3.6 FTU); and
Treatment 5 - SPEZYME XTRA (4 AAU).

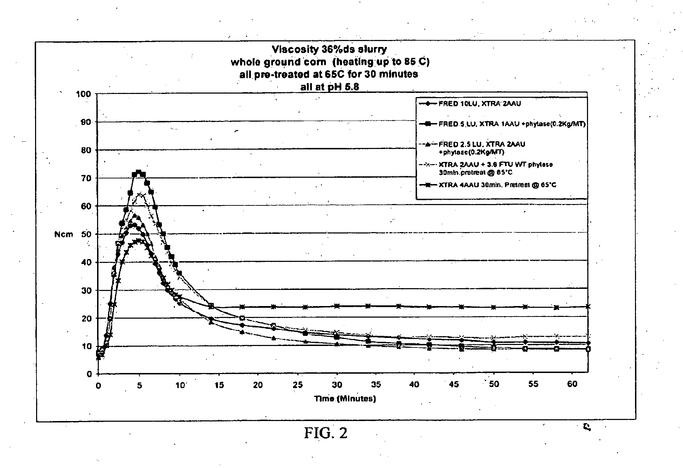

The results are illustrated in Figure 2. The addition of phytase extended the stability of the alpha amylase and this was supported by the continuous drop in viscosity (Ncm) (Y-axis) over time (X-axis) in Figure 2. At peak viscosity (about 72 to 75°C), SPEZYME XTRA was very effective in reducing viscosity but at higher temperatures the combination of SPEZYME FRED; SPEZYME XTRA and BP-WT was better at reducing viscosity.

### Example 9: Effect on viscosity of SPEZYME XTRA and BP-17

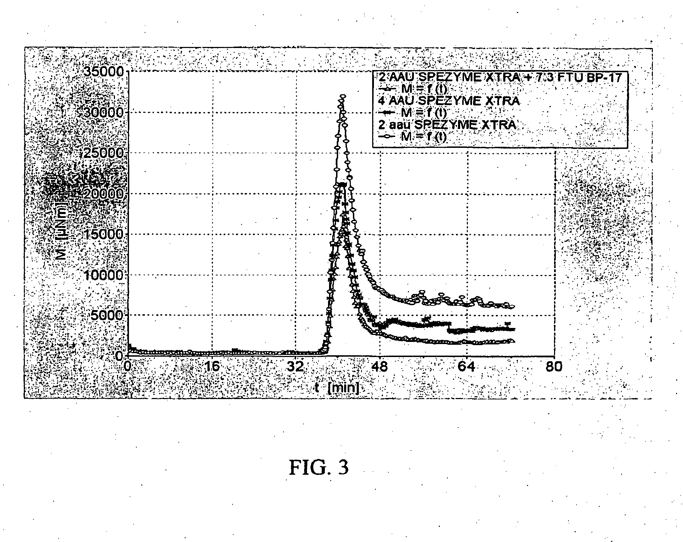

A 30% ds corn flour slurry (pH 5.8) was preincubated at 65°C for 10 min without enzyme and then incubated at 65°C for 30 min in the presence of SPEZYME XTRA (2.0 AAU/g and 4.0 AAU/g) without phytase or SPEZYME XTRA (2.0 AAU/g) + BP-17 (7.3 FTU/g). After 30 min of pretreatment with enzyme the temperature was increased to 85°C and the slurry was held at 85°C for an additional 30 minutes. As illustrated in Fig. 3 when viscosity (M (uNm) was measured over time (min) the addition of BP-17 reduced the amount of alpha amylase that was required to decrease viscosity of the slurry.

### Example 10: Effect of phytase on ethanol yields and DDGS

The effect of phytase on ethanol yields and DDGS was analyzed during a conventional liquefaction process. Liquefacts with and without phytase treatment were used in conventional yeast fermentations to compare the composition of DDGS for phytic acid and also to compare ethanol yields. Whole ground corn slurry, 32% ds corn containing 30% thin stillage VN was used, the pH was adjusted to pH 5.8 using dilute sodium hydroxide, and SPEZYME XTRA was added at 4 AAU/gds corn and incubated at 70°C for 30 min. The treated slurry was then passed through a pilot plant jet cooker maintained at 225°F with a hold time of 3 min. The gelatinized starch was then flashed to atmospheric pressure and held at 85°C. An additional dose of SPEZYME XTRA was added at 1.5 AAU/gds corn for completing the liquefaction and held for additional 90 min. Phytase treated liquefact was prepared, but BP-17 phytase was added at 4 FTU/gds corn during slurry treatment.

The pH of the liquefacts was then adjusted to pH 4.2 using dilute sulphuric acid and subjected to yeast fermentations. In each experiment tare weights of the vessels were obtained prior to preparation of media. 800 grams of a 32% DS corn liquefact were put in a 1 L flask. Red Star Ethanol Red yeast inoculum was prepared by adding 10 grams of yeast and 1 gram of glucose to 40 grams of water under mild agitation for one hour. Five films of yeast inoculums was added to equilibrated fermentors. G Zyme™ 480 Ethanol (Genencor - Danisco) was added at 0.4 GAU/ gds.corn to initiate the simultaneous saccharification and fermentation. The initial gross weight of the fermentation flask was noted and fermentor was placed in a water bath maintained at 32°C. Fermentations were carried out and weight loss during fermentation was measured at different intervals of time. The weight loss due to loss of carbon dioxide was used to calculate the alcohol yield. At the conclusion of the fermentation a final gross weight was obtained. The broth was quantitatively transferred into a 5L round bottom vessel. Distillation was performed under vacuum until approximately 800 mls of distillate was collected in a receptacle containing 200 mls water. The ethanol was diluted to 2L and was analyzed by HPLC. The weight and DS of the still bottoms was obtained prior to drying. Residual starch and Phytic acid analysis were performed on the DDGS and thin stillage. Stoichiometric calculations were performed based on weight loss, distillation, and residual starch analysis as follows:

Ethanol calculation using CO₂ weight loss:
Ethanol production (mmol) = CO₂ loss (g) / 88
Ethanol production (g) = (CO₂ loss (g) / 88) * 92 => CO₂ loss (g) * 1.045
Ethanol production (ml) = ((CO₂ loss (g) / 88) * 92) / 0.789
=> CO₂ loss (g) x 1.325

**Table 12: Comparison of DDGS from conventional liquefaction process and DDGS from a conventional process using SPEZYME XTRA and SPEZYME XTRA -BP-17 Phytase**

| Liquefaction conditions | Alcohol yield weight loss | Phytic acid DDGS (% ds) | Phytic acid Thin Stillage |
|---|---|---|---|
| Conventional Process-SPEZYME XTRA | 2.71 Gallon/Bushel | 1.21 | 480 ppm |
| Conventional Process-SPEZYME XTRA and BP-17 Phytase (PALS Process) | 2.69 Gallon /Bushel | 0.1-0.2 | 48 ppm |

The data in Table 12 showed major differences in phytic acid content in DDGS and thin stillage. Use of BP-17 resulted in more than 90% reduction of phytic acid in DDGS and thin stillage.

In examples 11-15, wildtype and variant *Buttiauxella* phytase were expressed directly or as a fusion protein in *Trichoderma reesei.* In all cases very strong levels of expression were seen at greater than 10g/L.

### Example 11 - Construction and Expression of the wildtype Buttiauxella Phytase in T. reesei as a Fusion Protein without a Kex2 site

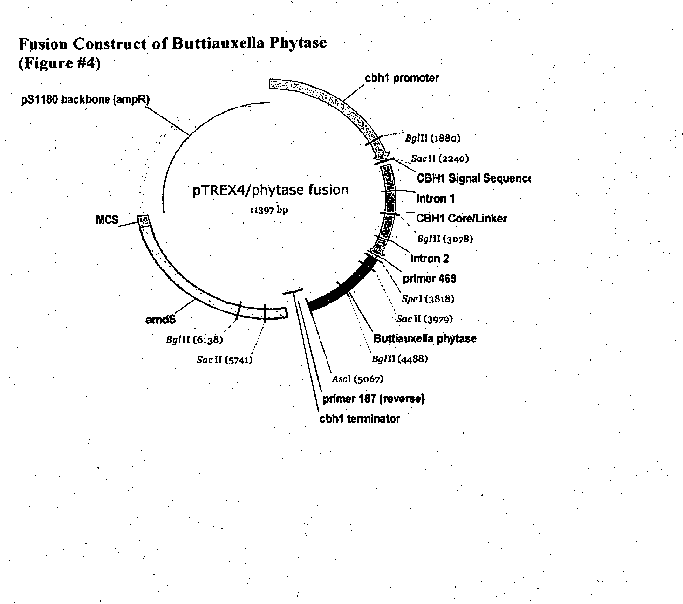

DNA encoding the wildtype *Buttiauxella* phytase open reading frame was synthesized by GENEART AG (BioPark Josef-Engert-Str. 11, D-93053 Regensburg, Germany). The restriction sites, SpeI and AscI were included for cloning proposes (see Table 13, SEQ ID NO:4). The phytase open reading frame (SEQ ID NO:4) was inserted into the vector, pTrex4, at the Spe1 and Asc1 sites (see Figure 4). The resulting construct was biolistically transformed into a strain derived from *T. reesei*, using the Biolistic PDS-1000/He Particle Delivery System from Bio-Rad (Hercules, CA). The transformation protocol used was as described by Foreman (WO 2005/001036). After stable transformants were obtained, these transformants were grown in shake flask cultures for expression analysis of the *Buttiauxella* phytase protein as outlined by Foreman (WO2005/001036). After several days of growth on MM acetamide plates, transformants displaying stable morphology were inoculated into 250 ml shake flasks containing 30 ml of Proflo medium. Proflo medium contained: 30 g/L α-lactose; 6.5 g/L (NH4)₂SO₄; 2g/L KH₂PO₄; 0.3 g/L MgSO₄.7H₂O; 0.2g/L CaCL₂; 1ml/L 1000X trace element salt solution; 2ml/L 10% Tween 80; 22.5g/L Proflo cottonseed flour (Traders Protein, Memphis, TN); and 0.72g/L CaCO₃. After two days of growth at 28°C and 225 rpm, 10% of the Proflo culture was transferred to a 250 ml shake flask containing 30 ml of Lactose Defined Media. The composition of Lactose Defined Media was as follows: 5g/L (NH₄)₂SO_{4;} 33g/L PIPPS buffer; 9g/L casamino acids; 4.5g/L KH₂PO_{4;} 1 g/L MgSO₄ 7H₂O; 5ml/L Mazu DF60-P antifoam (mazur Chemicals, Gurnee, IL); 1ml/L 1000X trace element salt solution; pH 5.5. 40ml/L of 40% (w/v) lactose solution was added to the medium after sterilization. The Lactose Defined medium shake flasks were incubated at 28°C, 225 rpm for 2-3 days. Samples of the culture supernatant were mixed with an appropriate volume of 4X NuPAGE sample buffer (Invitrogen Carlsbad, CA) with reducing agent and subjected to polyacrylamide gel electrophoresis (PAGE) using 4-12% NuPAGE precast gels, and MOPS running buffer (Invitrogen Carlsbad, CA). The gels were stained for protein detection with Simply Blue Stain (Invitrogen Carlsbad, CA). A protein band with an apparent molecular mass of approximately 96 kDa was observed on the stained gel. The expected molecular mass of the fusion protein is approximately 96 kDa. The protein was found to be expressed at greater than 10 g/L.

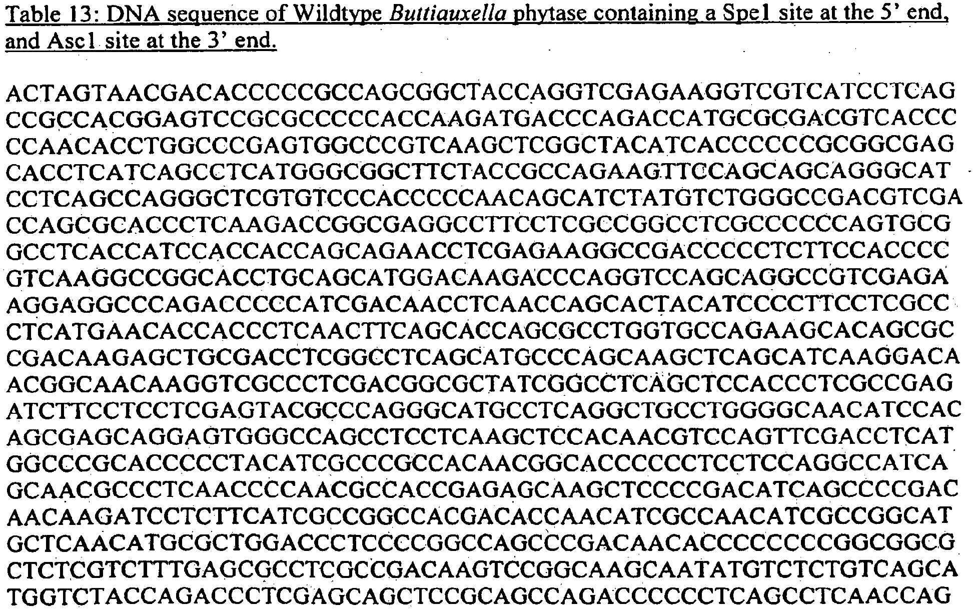

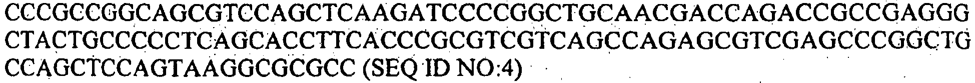

### Example 12 - Construction and Expression of the wildtype Buttiauxella Phytase in T. reesei as a Fusion Protein with a Kex2 site

The open reading frame of wildtype *Buttiauxella* phytase was amplified by polymerase chain reaction (PCR) using the DNA synthesized by GENEART as the template (see Table 13, SEQ ID NO:4). The PCR machine used was a Peltier Thermal Cycler PTC-200 (MJ Research). The DNA polymerase used in the PCR was HERculase (Stratagene). The primers used to amplify the phytase open reading frame were primer SK667 (forward) 5' CACTACTAGTGTCGCTGTGGAGAAGCGCAACGACACCCCCGCCAG-3' (SEQ ID NO:6), and primer SK664 5' GAGTTCGGCGCGCCTTACTGGA-3' (SEQ ID NO:7), The forward primer contained the amino acid sequence VAVEKR (SEQ ID NO:8) for efficient cleavage by the Kex2 protease, along with a SpeI site for cloning purposes. The PCR conditions for amplifying the wildtype *Buttiauxella* phytase open reading frame were as follows: Step1: 94°C for 1 min. Step 2: 94°C for 30 sec. Step 3: 58°C for 30 sec. Step 4: 72°C for 1 min. Steps 2, 3, and 4 were repeated for an additional 24 cycles. Step 5: 72°C for 5 min. Step 6: 4°C for storage. The PCR product was purified using Qiaquick Gel Purification Kit (Qiagen), and digested with restriction enzymes SpeI and AscI (Roche). The digested DNA was purified using Qiaquick PCR Purification Kit, and ligated into the pTrex4 vector at the SpeI and AscI sites (see Figure 4). The ligation reaction was transformed into TOP 10 chemically competent *E. coli* cells (Invitrogen). The resulting construct was biolistically transformed into a strain derived from *T. reesei*, using Biolistic PDS-1000/He Particle Delivery System from Bio-Rad (Hercules, CA). The transformation protocol used was that described by Foreman (WO 2005/001036). After stable transformants were obtained, these transformants were grown and protein expression identified as described in example 11. A protein band with an apparent molecular mass of approximately 96 kDa was observed on the stained gel. The expected molecular mass of the fusion protein is approximately 96 kDa. The protein was expressed at greater than 10 g/L.

### Example 13 - Construction and Expression of the Wildtype Buttiauxella Phytase in T. reesei as a Direct Construct

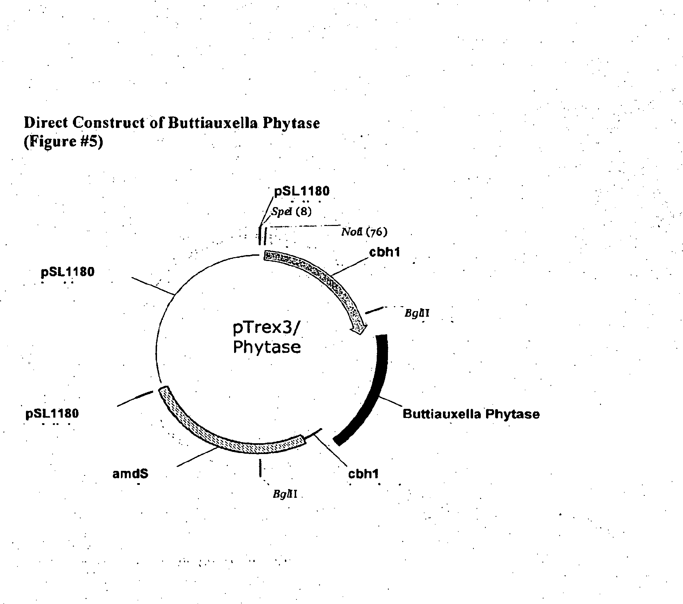

The open reading frame of wildtype *Buttiauxella* phytase was amplified by polymerase chain reaction (PCR) using the DNA synthesized by GENE ART as the template (see Table 13, SEQ ID NO:4). The PCR machine used was a Peltier Thermal Cycler PTC-200 (MJ Research). The DNA polymerase used in the PCR was HERculase (Stratagene). The primers used to amplify the phytase open reading frame were primer SK680 (forward) 5'-CACCATGCAGACCTTCGGTGCTTTTCTCGTTTCCTTCCTCGCCGCCAGCGGCCTGGCC GCGGCCAACGACACCCCCGCCAGC -3'(SEQ ID NO:9), and primer SK6 5'-CCTTACTGGAGCTGGCAG -3' (SEQ ID NO:10). The forward primer contained an additional four nucleotides (sequence - CACC) at the 5' end that was required for cloning into the pENTRY/D-TOPO vector (Invitrogen). The PCR conditions for amplifying the wildtype . *Buttiauxella* phytase open reading frame were as follows: Step1: 94°C for 1 min. Step2: 94°C for 30 sec. Step 3: 58°C for 30 sec. Step 4: 72°C for 1 min. Steps 2, 3, and 4 were repeated for an additional 24 cycles. Step 5: 72°C for 5 min. Step 6: 4°C for storage. The PCR product was purified using Qiaquick Gel Purification Kit (Qiagen). The purified PCR product was initially cloned into the pENTRY/D TOPO vector(Invitrogen), and transformed into TOP 10 chemically competent *E. coli* cells (Invitrogen). A pENTR/D-TOPO vector with the correct sequence of the phytase open reading frame was recombined with the pTrex3g vector using LR clonase II (Invitrogen) according to the manufacturers instructions (see Figure 5). The resulting construct was transformed and protein identified as described in example 11. A protein band with an apparent molecular mass of approximately 46 kDa was observed on the stained gel. The expected molecular mass of the fusion protein is approximately 46 kDa. The protein was expressed at greater than 10 g/L.

### Example 14 - Construction and Expression of the BP-17 variant Buttiauxella Phytase in T. reesei as a Fusion Protein with a Kex2 site

DNA encoding the BP-17 variant *Buttiauxella* phytase open reading frame was synthesized by GENEART AG (BioPark Josef-Engert-Str. 11, D-93053 Regensburg, Germany) (see Table 14, SEQ ID NO:5). The amino acid sequence VAVEKR (SEQ ID NO:8) was included for Kex2 protease cleavage of the fusion protein, along restriction sites SpeI and AscI for cloning proposes. The phytase open reading frame was inserted into the vector, pTrex4, at the Spe1 and Asc1 sites (see Figure 4). The resulting construct was transformed and protein identified as described in example 11. A protein band with an apparent molecular mass of approximately 96 kDa was observed on the stained gel. The expected molecular mass of the fusion protein is approximately 96 kDa. The protein was expressed at greater than 10 g/L.

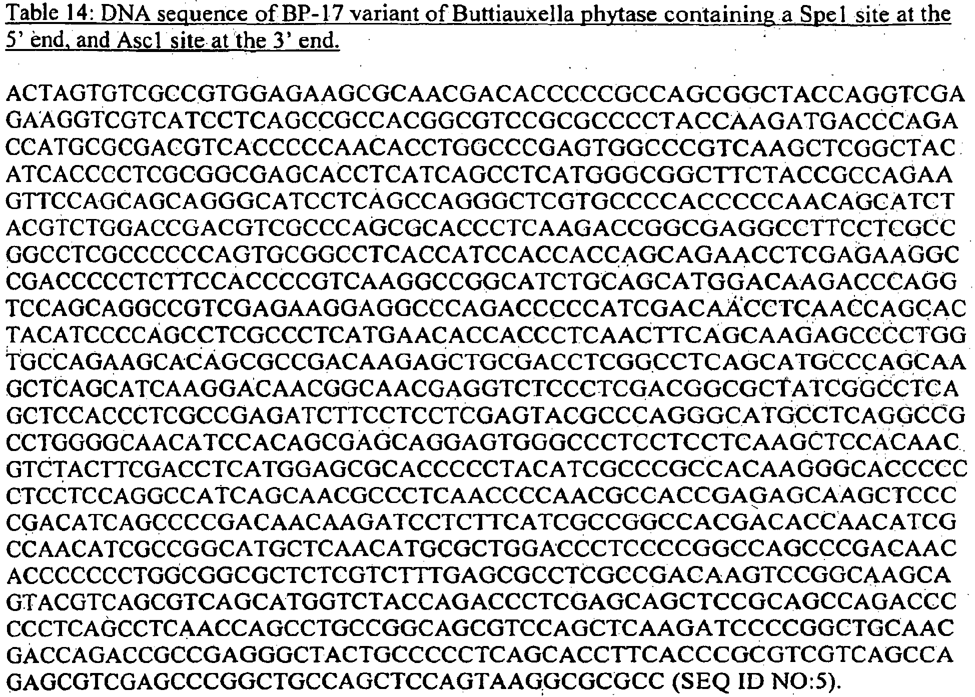

### Example 15 - Construction and Expression of the BP-17 variant Buttiauxella Phytase in T. reesei as a Direct Construct

The open reading frame of BP-17 variant *Buttiauxella* phytase was amplified by polymerase chain reaction (PCR) using the DNA synthesized by GENEART as the template (see Table 14, SEQ ID NO:5). The PCR machine used was a Peltier Thermal Cycler PTC-200 (MJ Research). The DNA polymerase used in the PCR was HERculase (Stratagene). The primers used to amplify the phytase open reading frame were primer SK680 (forward) 5'-CACCATGCAGACCTTCGGTGCTTTTCTCGTTTCCTTCCTCGCCGCCAGCGGCCTGGCC GCGGCCAACGACACCCCCGCCAGC -3' (SEQ ID NO:9), and primer SK6 5'-CCTTACTGGAGCTGGCAG -3'(SEQ ID NO:10). The forward primer contained an additional four nucleotides (sequence - CACC ) at the 5' end that was required for cloning into the pENTRY/D-TOPO vector (Invitrogen). The PCR conditions for amplifying the wildtype *Buttiauxella* phytase open reading frame were as follows: Step1: 94°C for 1 min. Step 2: 94°C for 30 sec. Step 3: 58°C for 30 sec. Step 4: 72°C for 1 min. Steps 2, 3, and 4 were repeated for an additional 24 cycles. Step 5: 72°C for 5 min. Step 6: 4°C for storage. The PCR product was purified using Qiaquick Gel Purification Kit (Qiagen). The purified PCR product was initially cloned into the pENTRY/D TOPO vector (Invitrogen), and transformed into TOP 10 chemically competent *E*. *coli* cells (Invitrogen) A pENTR/D-TOPO vector with the correct sequence of the phytase open reading frame was recombined with the pTrex3g vector using LR clonase II (Invitrogen) according to the manufacturer's instructions (see Figure 5). The resulting construct was transformed and protein expression identified as described in example 11. The Simply Blue staining analysis resulted in the observation of a protein band of approximately 46 kDa. A protein band with an apparent molecular mass of approximately 46 kDa was observed on the stained gel. The expected molecular mass of the fusion protein is approximately 46 kDa. The protein was found to be expressed at greater than 10 g/L.

Examples 16-20 provide more data showing that the combination of an alpha amylase and a *Buttiauxella* phytase reduces the inhibition of the alpha amylase and allows ethanol fermentation to proceed at a lower pH, even without addition of alkali or acid.

### EXAMPLE 16: Effect of removal of phytic acid inhibition on alpha amylase thermostability

An aqueous slurry of whole ground corn (32-40 % ds corn containing 50 % thin stillage) was incubated at pH 5.8, 70°C. A thermostable phytase (BP-17) and two liquefying thermostable alpha amylases SPEZYME™ XTRA and SPEZYME™ ETHYL from Danisco US Inc, Genencor Division, were used in the studies for comparison

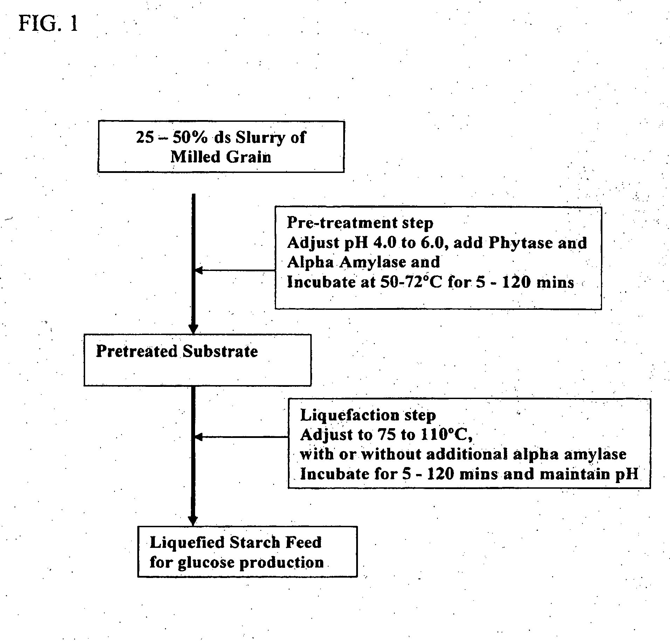

Whole ground corn (Badger State Ethanol, Monroe, WI) was mixed with water containing 50% (VN) thin stillage to a final concentration of 32% ds. Corn solids were prepared in a jacked kettle. The slurry was then mixed well and the pH of the slurry was adjusted to pH 5.8 which is a typical pH for the liquefaction of a commercial ethanol process using sodium carbonate or sodium hydroxide. This slurry was mixed in a jacketed kettle and brought up to the pretreatment temperature of 65-70°C. Just prior to reaching 70°C, the liquefying enzymes SPEZYME™ XTRA (10 AAU per gram ds corn) or genetically modified alpha amylase from *Bacillus stearothermophilus* (SPEZYME™ ETHYL, from Danisco US Inc, Genencor Division) were added and a timer was started to begin the primary liquefaction step (1° liquefaction, see Figure 1). The slurry was incubated for 40 minutes in the presence of the enzymes with or without added BP-17 phytase (12 FTU per gram ds corn). The pretreated slurry was then passed through a jet cooker (82-107°C, 180-225° F) which was preheated to the desired temperature using steam and water. The slurry was sent through the jet at maximum speed (1.5 setting) about 4 liters/minute. Using the first three loops of the hold coil resulted in a hold time of just over 3 minutes. After all of the water was displaced and the desired temperature held steady, an aliquot of solubilized corn mash was collected and placed in a secondary bath (overhead stirring) at 85°C to begin the secondary liquefaction step (2° liquefaction). Samples were taken to test for viscosity (by Brookfield), brix and DE (by Schoorls) at 0, 30, 60 and 90 minutes. The results are summarized in Table 15.

Table 15 shows a comparison of the effect of primary liquefaction conditions on the thermostability of different alpha amylases after passing though jet cooking conditions (225°F) for whole ground corn. The primary liquefaction conditions were: 32% whole ground corn slurry in water, pH adjusted to pH 5.8, incubated at 70°C for 40 min in the presence of different enzymes. Jet cooking conditions were at 107°C(225°F) for 3 min.

**Table 15:**

| Enzyme Treatment | Phytase (BP-17) in the 1° liquefaction | Time @85°C | DE | Viscosity,CPS |
|---|---|---|---|---|
| SPEZYME™ XTRA 10 AU/g ds.corn,32% ds corn slurry containing 50 % thin stillage, pH 5.8 | | 0 | 9.56 | 6840 |
| | No | 30 min | 9.41 | 9900 |
| | | 60 min | 9.95 | 9880 |
| | | 90 min | 9.78 | 9800 |
| SPEZYME™ ETHYL 10AAU/g ds.com,32% ds corn slurry containing 50 % thin stillage, pH 5.8 | No | 0 | 7.55 | 5060 |
| | | 30 min | 7.88 | 4340 |
| | | 60 min | 8.15 | 4240 |
| | | 90 min | 8.44 | 3750 |
| SPEZYME™ XTRA 10AAU/g ds corn+ 12 FTU, BP-17Phytase/g ds corn | Yes | 8.27 | 11.04 | 1060 |
| | | 30 min | 15.73 | 700 |
| pH 5.8 | | 60 min | 16.84 | 750 |
| | | 90 min | 17.9 | 750 |

Addition of BP-17 phytase during primary liquefaction reduced the phytic acid content of the whole ground corn from 0.60 % ds com to 0.09 % ds corn (> 85 % reduction). The data in Table 15 showed that SPEZYME™ XTRA and SPEZYME™ ETHYL were completely inactivated at a jet cooking temperature of 107°C(225°F) based on DE development or viscosity reduction. However, the removal of phytic acid inhibition by phytase prior to jet cooking resulted in a significant increase in the thermostability of the alpha amylases as shown by DE progression and viscosity reduction at 85°C during the secondary liquefaction step. The results showed that the phytic acid inhibited alpha amylases and that removal of the inhibition increased the thermostability and/or pH stability of liquefying thermostable alpha amylase.

### EXAMPLE 17: Effect of removal of phytic acid inhibition on alpha amylase pH stability

Whole ground corn was slurried to a 32% (ds corn) slurry by using a 50:50 ratio of water and thin stillage. The slurry pH was measured and found to be pH 5.15. The slurry was heated to 70°C (158°F) using water and steam in a jacketed kettle. The liquefaction enzymes (SPEZYME XTRA and BP-17) were added and the slurry was pretreated by holding the temperature at 70°C for 40 minutes. After 40 minutes of pretreatment the slurry was passed through a jet-cooker maintained at 107°C(225°F) with a 3 minute hold time using a large pilot plant jet (equipped with an M103 hydro-heater). The liquefact was collected from the jet and placed in an 85°C water bath. The liquefact was continuously stirred and held at 85°C for 90 minutes. Samples were collected at 0, 30, 60 and 90 minutes. All samples were tested for Brix, DE (using the Schoorls method), and for viscosity (Brookfield viscometer spindle 2 at 20 rpms). The liquefaction studies were also conducted using SPEZYME™ ETHYL and BP-17 phytase.

Table 16 shows the DE progression and viscosity reduction during liquefaction of whole ground corn without any pH adjustment. The data showed that SPEZYME™ XTRA or SPEZYME™ ETHYL can be successfully used in the liquefaction process for whole ground corn at a pH of 5.2 if the inhibition of the alpha amylase by phytic acid is eliminated.

**Table 16:**

| Enzyme Treatment | Phytase (BP-17) 1° liquefaction step (40 m 70 °C) | %Phytic acid removed | Time@85°C, | DE | Viscosity, CPS |
|---|---|---|---|---|---|
| SPEZYME™ XTRA 10AAU/g ds.corn,32% ds corn slurry containing 50 % thin stillage, pH 5.15 | 12.8 FTU/g ds corn | | 0 | 10.38 | 3620 |
| | | | 30 min | 12.69 | 1630 |
| | | | 60 min | 14.69 | 1740 |
| | | | 90 min | 15.62 | 2140 |
| SPEZYME™ ETHYL 10AAU/g ds.corn,32% ds corn slurry containing 50 % thin stillage, pH 5.15 | 12.8 FTU/g ds corn | | 0 | 8.38 | 2200 |
| | | | 30 min | 9.78 | 1280 |
| | | | 60 min | 11.70 | 1250 |
| | | | 90 min | 12.54 | 1290 |

The results in Table 15 and Table 16 showed that the reduction of phytic acid inhibition of SPEZYME™ XTRA and SPEZYME™ ETHYL prior to high temperature jet cooking at 107°C(225°F) of whole ground corn resulted in a significant increase in the stability of the activity at low pH stability as evidenced by a steady increase in the DE progression at 85°C with a concomitant decrease in the viscosity of the liquefact.

### EXAMPLE 18: Single dose versus split dose alpha amylase,

This example illustrates the comparison of single dose and split dose addition of alpha amylase in the liquefaction process of whole ground corn. Whole ground corn was slurried to a 40%(ds corn) using water and thin stillage (2.8% of total). This slurry was then pH adjusted to 5.2 using 6N sulfuric acid. The slurries were heated to 68°C(155°F) using water and steam in a jacketed kettle. SPEZYME XTRA and BP-17 phytase were added at 10 AAU/g ds corn and 12.8 FTU/g ds corn respectively. The slurry was pretreated by holding the temperature at 68°C(155°F) for 40 minutes. After 40 minutes of pretreatment the slurry was passed through a jet cooker maintained at 107°C(225°F) with one minute hold time using a pilot plant jet (equipped with an M103 hydro-heater). The liquefact was collected from the jet and placed in an 85°C water bath for secondary liquefaction. Three separate secondary liquefactions were carried out, 1) no additional SPEZYME XTRA, 2) an additional 1 AAU/g ds corn dose of alpha amylase, and 3) dosed with an additional 2 AAU/g ds corn of alpha amylase. The liquefact was continuously stirred and held at 85°C for 90 minutes. Samples were collected at 0, 35 and 60 minutes. All samples were tested for Brix, DE (using the Schoorls method), and for viscosity (Brookfield viscometer spindle 2 at 20 rpms) Table 17.

Table 17 is a comparison of single dose (primary liquefaction) and split dose (secondary liquefaction) SPEZYME™ XTRA.

**Table 17:**

| Conditions for 1° Liquefaction | SPEZYME™ XTRA addition at the 2° Liquefaction | Time@85°C | DE | Viscosity, CPS |
|---|---|---|---|---|
| SPEZYME XTRA at 10 AAU/g ds.corn, + BP-17 Phytase at 12.0 FTU/g ds corn 40% ds corn slurry containing 2.8 % thin stillage pH 5.15. Incubated 70° C for 40 min | No addition | 0 | 11.87 | 3220 |
| | **Single Dose** | 35min | 14.63 | 1440 |
| | | 60 min | 15.36 | 1330 |
| | 1 AAU/g ds corn | 0 | 11.87 | 3220 |
| | **Split Dose** | 35min | 15.78 | 1130 |
| | | 60 min | 16.75 | 1170 |
| | 2AAU/g ds corn | 0 | 11.87 | 3220 |
| | **Split Dose** | 35 min | 16.77 | 860 |
| | | 60 min | 17.69 | 1040 |

The data in Table 17 showed that a significant amount of SPEZYME™ XTRA activity survived through the jet cooking temperature of 107°C(225°F) due to the stabilization of SPEZYME™ XTRA in the primary liquefaction step. Both increases in DE and viscosity reduction were seen without the addition of the second dose of SPEZYME™ XTRA at the secondary liquefaction step. However, addition of a second dose of SPEZYME™ XTRA at the secondary liquefaction step further enhanced the DE progression as well as viscosity reduction.

### EXAMPLE 19: Effect on DDGS and ethanol production

Liquefacts were used as fermentation feedstocks in ethanol fermentation for alcohol production. The liquefact #1 (32% ds corn containing 50 % thin stillage) from a conventional liquefaction process using SPEZYME™ XTRA at pH 5.8 without phytase in the primary liquefaction step was used. Also used was the liquefact from Example 17, liquefact #2, using SPEZYME™ XTRA with phytase treatment in the primary liquefaction step and with no pH adjustment made prior to fermentation. The pH of the control liquefact-1 was adjusted to 4.2 using dilute sulfuric acid as in the conventional ethanol process whereas the liquefact from Example 17 was used (liquefact #2) without any further pH adjustment. The liquefact from Example 17 was used as the no pH adjustment test for the process of the present invention. In each experiment tare weights of the vessels were obtained prior to preparation of media. A 32% DS corn liquefact (2 liters) was taken in a 2 L flask. Red Star Ethanol Red yeast (RED STAR (Lesaffre), inoculums were prepared by adding 10 grams of yeast and 1 gram of glucose to 40 grams of water under mild agitation for one hour. Five mls of each inoculum was added to equilibrated fermentors followed by the addition of G Zyme™ 480 Ethanol (Danisco US Inc, Genencor Division) at 0.4 GAU/ g d corn to initiate the simultaneous saccharification and fermentation. The initial gross weight was noted and the flask was placed in a water bath maintained at 32°C. The samples were taken at different intervals of time and analyzed for carbohydrate and ethanol content using HPLC. Fermentations were also carried out using one kilogram of each liquefact and weight loss during fermentation was measured at different intervals of time. Based on the weight loss due to loss of carbon dioxide, the alcohol was measured (Table 18). At the conclusion of the fermentation, a final gross weight was obtained. The broth was quantitatively transferred into a 5L round bottom vessel. Distillation was performed under vacuum until approximately 800 mls of ethanol was collected in a receptacle containing 200 mls water. The ethanol was diluted to 2L and was analyzed by HPLC. The weight and DS of the still bottoms was obtained prior to drying. Residual starch analysis was performed on the DDGS. Stoichiometric calculations were performed based on weight loss, distillation, and residual starch analysis.

Ethanol calculation using CO₂ weight loss:
Ethanol production (mmol) CO₂ loss (g) / 88
Ethanol production (g) = (CO₂ loss (g) / 88) * 92 => CO₂ loss (g) * 1.045
Ethanol production (ml) = ((CO₂ loss (g) / 88) * 92) / 0.789 => CO₂ loss (g) x 1.325

**Table 18: Comparison of DDGS from conventional liquefaction process and DDGS from No pH adjustment process.**

| Liquefaction conditions | Alcohol yield weight loss | DDGS,% ds | | | | |
|---|---|---|---|---|---|---|
| | | Starch | Phytic acid | % IP 6 | Free Phosphate | Sulfate (mg/gds) |
| Conventional Process-pH 5.8 (liquefact #1) | 2.70 Gallon/Bushel | 7.25 | 0.6. | 100 | 1.20 | 1.92 |
| No pH adjustment, pH 5.2 (liquefact #2) | 2.69 Gallon/Bushel | 9.28 | 0.2 | 0 | 1.33 | 0.23 |

The data in Table 18 showed major differences in free sulphate and phytic acid content between the processes. Removal of phytic acid inhibition of thermostable alpha amylase in the primary liquefaction resulted in DDGS with reduced phytic acid content, higher free available phosphate and reduced sulfate. Thus, the process with no pH adjustment conferred pH stability at low pH for liquefying thermostable alpha amylases in the starch liquefaction process.

## Claims

1. A method for liquefying starch, said method comprising:
(a) incubating a slurry comprising a granular starch substrate with an enzyme composition, at a temperature which is above 55°C and below the initial starch gelatinization temperature of the granular starch substrate, for about 2 mins to about 4 hrs at a pH range of about 4.0 to about 6.2;
(b) raising the temperature to 0 to about 45°C above the initial starch gelatinization temperature for about 5 mins to about 6 hrs at a pH of between about pH 4.0 and about 6.2 and obtaining liquefied starch;
wherein the enzyme composition comprises a mixture of a phytase and an alpha amylase, wherein the phytase has an amino acid sequence having at least 90% sequence identity to the sequence BP-WT: 
NDTPASGYQV EKVVILSRHG VRAPTKMTQT MRDVTPNTWP EWPVKLGYIT
PRGEHLISLM GGFYRQKFQQ QGILSQGSCP TPNSIYVWAD VDQRTLKTGE
AFLAGLAPQC GLTIHHQQNL EKADPLFHPV KAGTCSMDKT QVQQAVEKEA
QTPIDNLNQH YIPFLALMNT TLNFSTSAWC QKHSADKSCD LGLSMPSKLS
IKDNGNKVAL DGAIGLSSTL AEIFLLEYAQ GMPQAAWGNI HSEQEWASLL
KLHNVQFDLM ARTPYIARHN GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 or to the sequence BP-11: 
NDTPASGYQV EKVVILSRHG VRAPTKMTQT MRDVTPNTWP EWPVKLGYIT
PRGEHLISLM GGFYRQKFQQ QGILSQGSCP TPNSIYVWTD VDQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 or to the sequence BP-17:

NDTPASGYQV EKVVILSRHG VRAPTKMTQT MRDVTPNTWP EWPVKLGYIT
PRGEHLISLM GGFYRQKFQQ QGILSQGSCP TPNSIYVWTD VAQRTLKTGE
AFLAGLAPQC GLTIHHQQNL EKADPLFHPV KAGICSMDKT QVQQAVEKEA

QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ.

2. The method of claim 1, wherein the alpha amylase is from a *Bacillus sp..*

3. The method of claim 2 wherein the *Bacillus* sp. is *Bacillus stearothermophilus* or *Bacillus licheniformis.*

4. The method of claim 1, wherein the composition is a starch hydrolyzing composition.

5. The method of claim 1 wherein the phytase has at least 95% sequence identity to the sequence BP-WT, BP-11 or BP-17.

6. The method of claim 1, wherein the phytase has the sequence of BP-11 or BP-17.

7. The method of claim 1, further comprising saccharifying the liquefied starch to obtain dextrins; and recovering the dextrins.

8. The method of claim 7, further comprising fermenting the dextrins under suitable fermentation conditions to obtain end-products.

9. The method of claim 8, wherein the end-products are selected from the group of:
alcohol, organic acids, sugar alcohols, ascorbic acid intermediates, amino acids, and proteins.

10. The method of claim 9 wherein the alcohol is ethanol.

## Patentansprüche

1. Verfahren zur Verflüssigung von Stärke, wobei das Verfahren umfasst:
(a) Inkubieren einer ein Stärkegranulatsubstrat umfassenden Aufschlämmung mit einer Enzymzusammensetzung, bei einer Temperatur, die über 55 °C und unter der Stärkeverkleisterungs-Anfangstemperatur des Stärkegranulatsubstrats liegt, etwa 2 min bis etwa 4 h lang bei einem pH-Wert im Bereich von etwa 4,0 bis etwa 6,2;
(b) Erhöhen der Temperatur auf 0 °C bis etwa 45 °C über der Stärkeverkleisterungs-Anfangstemperatur etwa 5 min bis etwa 6 h lang bei einem pH-Wert zwischen etwa pH 4,0 und etwa 6,2, und Erhalten verflüssigter Stärke;
wobei die Enzymzusammensetzung ein Gemisch von einer Phytase und einer alpha-Amylase umfasst, wobei die Phytase eine Aminosäuresequenz aufweist mit zumindest 90% Sequenzidentität zur Sequenz BP-WT: 
NDTPASGYQV EKVVILSRHG VRAPTKMTQT MRDVTPNTWP EWPVKLGYIT
PRGEHLISLM GGFYRQKFQQ QGILSQGSCP TPNSIYVWAD VDQRTLKTGE
AFLAGLAPQC GLTIHHQQNL EKADPLFHPV KAGTCSMDKT QVQQAVEKEA
QTPIDNLNQH YIPFLALMNT TLNFSTSAWC QKHSADKSCD LGLSMPSKLS
IKDNGNKVAL DGAIGLSSTL AEIFLLEYAQ GMPQAAWGNI HSEQEWASLL
KLHNVQFDLM ARTPYIARHN GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 oder zur Sequenz BP-11: 
NDTPASGYQV EKVVILSRHG VRAPTKMTQT MRDVTPNTWP EWPVKLGYIT
PRGEHLISLM GGFYRQKFQQ QGILSQGSCP TPNSIYVWTD VDQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 oder zur Sequenz BP-17:

NDTPASGYQV EKVVILSRHG VRAPTKMTQT MRDVTPNTWP EWPVKLGYIT
PRGEHLISLM GGFYRQKFQQ QGILSQGSCP TPNSIYVWTD VAQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ.

2. Verfahren nach Anspruch 1, wobei die alpha-Amylase von einer *Bacillars Sp.* ist.

3. Verfahren nach Anspruch 2, wobei die *Bacillus Sp. Bacillus stearothermophilus* oder *Bacillus licheniformis* ist.

4. Verfahren nach Anspruch 1, wobei die Zusammensetzung eine Stärke hydrolysierende Zusammensetzung ist.

5. Verfahren nach Anspruch 1, wobei die Phytase zumindest 95% Sequenzidentität zur Sequenz BP-WT, BP-11 oder BP-17 aufweist.

6. Verfahren nach Anspruch 1, wobei die Phytase die Sequenz von BP-11 oder BP-17 aufweist.

7. Verfahren nach Anspruch 1, weiterhin umfassend Verzuckern der verflüssigten Stärke, um Dextrine zu erhalten; und Gewinnen der Dextrine.

8. Verfahren nach Anspruch 7, weiterhin umfassend Gären der Dextrine unter geeigneten Gärungsbedingungen, um Endprodukte zu erhalten.

9. Verfahren nach Anspruch 8, wobei die Endprodukte gewählt sind aus der Gruppe:
Alkohol, organische Säuren, Zuckeralkohole, Ascorbinsäure-Zwischenprodukte, Aminosäuren und Proteine.

10. Verfahren nach Anspruch 9, wobei der Alkohol Ethanol ist.

## Revendications

1. Procédé de liquéfaction d'amidon, ledit procédé comprenant:
(a) l'incubation d'une suspension comprenant un substrat granulaire d'amidon avec une composition d'enzyme, à une température qui est supérieure à 55°C et inférieure à la température de gélatinisation initiale de l'amidon du substrat granulaire d'amidon, durant environ 2 min à environ 4 h à une plage de pH d'environ 4,0 à environ 6,2;
(b) l'élévation de la température à 0 à environ 45°C au-dessus de la température de gélatinisation initiale de l'amidon durant environ 5 min à environ 6 h à un pH compris entre environ le pH 4,0 et environ 6,2 et l'obtention d'amidon liquéfié;
où la composition d'enzyme comprend un mélange d'une phytase et d'une alpha amylase, où la phytase a une séquence d'acides aminés ayant au moins 90 % d'identité de séquence avec la séquence BP-WT: 
NDTPASGYQV EKVVILSRHG VRAPTKMTQT MRDVTPNTWP EWPVKLGYIT
PRGEHLISLM GGFYRQKFQQ QGILSQGSCP TPNSIYVWAD VDQRTLKTGE
AFLAGLAPQC GLTIHHQQNL EKADPLFHPV KAGTCSMDKT QVQQAVEKEA
QTPIDNLNQH YIPFLALMNT TLNFSTSAWC QKHSADKSCD LGLSMPSKLS
IKDNGNKVAL DGAIGLSSTL AEIFLLEYAQ GMPQAAWGNI HSEQEWASLL
KLHNVQFDLM ARTPYIARHN GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 ou la séquence BP-11: 
NDTPASGYQV EKVVILSRHG VRAPTKMTQT MRDVTPNTWP EWPVKLGYIT
PRGEHLISLM GGFYRQKFQQ QGILSQGSCP TPNSIYVWTD VDQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ
 ou la séquence BP-17:

NDTPASGYQV EKVVILSRHG VRAPTKMTQT MRDVTPNTWP EWPVKLGYIT
PRGEHLISLM GGFYRQKFQQ QGILSQGSCP TPNSIYVWTD VAQRTLKTGE
AFLAGLAPQC GLTIHHQQNL EKADPLFHPV KAGICSMDKT QVQQAVEKEA
QTPIDNLNQH YIPSLALMNT TLNFSKSPWC QKHSADKSCD LGLSMPSKLS
IKDNGNEVSL DGAIGLSSTL AEIFLLEYAQ GMPQAAWGNI HSEQEWALLL
KLHNVYFDLM ERTPYIARHK GTPLLQAISN ALNPNATESK LPDISPDNKI
LFIAGHDTNI ANIAGMLNMR WTLPGQPDNT PPGGALVFER LADKSGKQYV
SVSMVYQTLE QLRSQTPLSL NQPAGSVQLK IPGCNDQTAE GYCPLSTFTR
VVSQSVEPGC QLQ.

2. Procédé selon la revendication 1, dans lequel l'alpha amylase provient de *Bacillus sp.*

3. Procédé selon la revendication 2 dans lequel le *Bacillus sp.* est *Bacillus stearothermophilus* ou *Bacillus licheniformis.*

4. Procédé selon la revendication 1, dans lequel la composition est une composition d'hydrolyse d'amidon.

5. Procédé selon la revendication 1, dans lequel la phytase a au moins 95 % d'identité de séquence par rapport à la séquence BP-WT, BP-11 ou BP-17.

6. Procédé selon la revendication 1, dans lequel la phytase a la séquence de BP-11 ou BP-17.

7. Procédé selon la revendication 1, comprenant en outre la saccharification de l'amidon liquéfié pour obtenir des dextrines; et la récupération des dextrines.

8. Procédé selon la revendication 7, comprenant en outre la fermentation des dextrines sous des conditions appropriées de fermentation pour obtenir des produits finaux.

9. Procédé selon la revendication 8, dans lequel les produits finaux sont sélectionnés parmi le groupe de: l'alcool, les acides organiques, les alcools de sucre, les intermédiaires d'acide ascorbique, les acides aminés, et les protéines.

10. Procédé selon la revendication 9 dans lequel l'alcool est l'éthanol.
